# EUROPEAN PATENT APPLICATION

(11) **EP 4 620 970 A1**
(43) Date of publication of application: **24.09.2025**
(21) Application number: 22969624.0
(22) Date of filing: 28.12.2022
(51) Int. Cl.: C07K 14/00, C12N 15/11, C12N 15/63, C12N 5/10, C12Q 1/6869

(54) **PORIN MONOMER, PORIN, MUTANT THEREOF, AND USE THEREOF**

(71) Applicant: BGI Shenzhen, Shenzhen, Guangdong 518083 (CN); BGI Shenzhen Co., Ltd, Shenzhen, Guangdong 518083 (CN)
(72) Inventor: WANG, Lele, Shenzhen, Guangdong 518083 (CN); LIU, Huanhuan, Shenzhen, Guangdong 518083 (CN); PENG, Fei, Shenzhen, Guangdong 518083 (CN); JIANG, Nan, Shenzhen, Guangdong 518083 (CN); WANG, Zi, Shenzhen, Guangdong 518083 (CN); CHEN, Junyi, Shenzhen, Guangdong 518083 (CN); JI, Zhouxiang, Shenzhen, Guangdong 518083 (CN); GUO, Fei, Shenzhen, Guangdong 518083 (CN); ZENG, Tao, Shenzhen, Guangdong 518083 (CN); LI, Yuxiang, Shenzhen, Guangdong 518083 (CN); DONG, Yuliang, Shenzhen, Guangdong 518083 (CN); ZHANG, Wenwei, Shenzhen, Guangdong 518083 (CN); XU, Xun, Shenzhen, Guangdong 518083 (CN)
(74) Representative: Delbarba, Andrea
(86) International application number: PCT/CN2022/143060
(87) International publication number: WO 2024/138473

(57) **Abstract**

The present application provides a porin monomer, a porin, and a mutant and application thereof. The porin monomer includes: (a) a protein having an amino acid sequence shown in SEQ ID NO: 1; or (b) a protein mutant, where an amino acid sequence of the protein mutant is subjected to substitution, deletion and/or addition of one or more amino acids at at least one of the following sites in SEQ ID NO: 1: 77, 81, 82, and the like, and the mutant has a function of forming a pore channel structure by means of polymerization; or (c) a protein having at least 70%, 75%, 80%, 85%, 90%, 95%, or 99% homology to the protein in the (a) or the (b), where the mutant has the function of forming the pore channel structure by means of polymerization. Therefore, the problem in the prior art of a low capture rate of a nano porin to a library can be solved, and the present application is applicable to the field of single-molecule sequencing.

## Description

### Technical Field

The present disclosure relates to the field of single-molecule sequencing, and specifically, to a porin monomer, a porin, and a mutant and application thereof.

### Background

At present, a number of commercially available nanopore sequencers have appeared on the market, for example, MinION, GridION, and PromethlON of Oxford Nanopore Technologies, and QNome-3841 of QITAN TECH. However, the nanopore sequencers still have significant deficiencies in sequencing accuracy and throughput, as well as chip stability and applicable scenarios, and thus cannot meet an ultimate requirement for molecular biology research. Therefore, it is urgent to develop a single-molecule sequencer with high accuracy, high integration, and high stability. The nanopore-based single-molecule sequencer is a highly integrated detection system with multiple disciplines and technologies. The development of such instruments requires in-depth interdisciplinary and collaborative innovation in physics, biology, chemistry, semiconductors, computers, etc., and a high-accuracy single-molecule nanopore sequencing system is constructed from an underlying core module.

Nanopore sequencing requires a sensing region inside a pore protein to be sharp enough to have high spatial resolution in both lateral and longitudinal directions. As of now, only few natural proteins such as Mycobacterium smegmatis porin A (MspA), Curli-specific transport channel (CsgG), etc. can industrially qualify as porins for single-molecule detectors. Finding more excellent porins that may be used for single-molecule sequencing through gene mining methods remains an open question.

### Summary

The present disclosure is mainly intended to provide a porin monomer, a porin, and a mutant and application thereof, so as to solve the problem in the prior art of a low capture rate of a nano porin to a library.

In order to implement the above objective, a first aspect of the present disclosure provides a porin monomer. The porin monomer includes: (a) a protein having an amino acid sequence shown in SEQ ID NO: 1; or (b) a protein mutant, where an amino acid sequence of the protein mutant is subjected to substitution, deletion and/or addition of one or more amino acids at at least one of the following sites in SEQ ID NO: 1: 77, 81, 82, 176, 210, 214, 232, 66, 69, 70, 74, 109, 110, 113, 117, 118, 119, 120, 123, 127, 128, 168, 211, 221, 224, 227, and 229, and the mutant has a function of forming a pore channel structure by means of polymerization; or (c) a protein having at least 70%, 75%, 80%, 85%, 90%, 95%, or 99% homology to the protein in the (a) or the (b), where the protein has the function of forming the pore channel structure by means of polymerization.

Further, in the (b), types of amino acids substituted are each independently selected as follows: S77 is mutated into S77G, S77A, or S77T; S81 is mutated into S81G, S81A, S81T, S81N, or S81Q; F82 is mutated into F82G, F82A, F82S, F82T, F82N, or F82Q; E176 is mutated into E176A, E176G, E176V, E176L, E176I, E176Y, E176F, or E176W; K210 is mutated into K210A, K210G, K210V, K210L, K210I, K210Y, K210F, or K210W; S214 is mutated into S214A, S214G, S214V, S214L, S214I, S214Y, S214F, or S214W; T232 is mutated into T232A, T232G, T232V, T232L, T232I, T232Y, T232F, or T232W; K66 is mutated into K66N, K66A, K66G, K66S, K66T, or K66Q; D69 is mutated into D69K, D69R, D69N, D69A, D69G, D69S, D69T, or D69Q; Q70 is mutated into Q70K, Q70R, Q70D, Q70E, Q70N, Q70A, Q70G, Q70S, Q70T, or Q70Q; Y74 is mutated into Y74N, Y74A, Y74G, Y74S, Y74T, or Y74Q; R109 is mutated into R109N, R109A, R109G, R109S, R109T, or R109Q; E110 is mutated into E110K, E110R, E110N, E110A, E110G, E110S, E110T, or E110Q; Q113 is mutated into Q113K, Q113R, Q113N, Q113A, Q113G, Q113S, or Q113T; T117 is mutated into T117K, T117R, T117N, T117A, T117G, T117S, or T117Q; E118 is mutated into E118K, E118R, E118N, E118A, E118G, E118S, E118T, or E118Q; R119 is mutated into R119N, R119A, R119G, R119S, R119T, or R119Q; K120 is mutated into K120N, K120A, K120G, K120S, K120T, or K120Q; R123 is mutated into R123N, R123A, R123G, R123S, R123T, or R123Q; K127 is mutated into K127N, K127A, K127G, K127S, K127T, or K127Q; K128 is mutated into K128N, K128A, K128G, K128S, K128T, or K128Q; R168 is mutated into R168N, R168Q, R168S, R168T, R168A, or R168G; E211 is mutated into E211N, E211Q, E211S, E211T, E211A, or E211G; E221 is mutated into E221N, E221Q, E221S, E221T, E221A, or E221G; E224 is mutated into E224N, E224Q, E224S, E224T, E224A, or E224G; E227 is mutated into E227N, E227Q, E227S, E227T, E227A, or E227G; and E229 is mutated into E229N, E229Q, E229S, E229T, E229A, or E229G.

Further, in the (b), the types of amino acids substituted are each independently selected as follows: S77A, F82Q, S81N, F82A, K120N, K127S, and E176I.

Further, the porin monomer includes a protein having an amino acid sequence in any one of SEQ ID NO: 2 to SEQ ID NO: 6.

In order to implement the above objective, a second aspect of the present disclosure provides a protein construct. The protein construct is formed by 2 or more porin monomers through a covalent or non-covalent linkage.

In order to implement the above objective, a third aspect of the present disclosure provides a porin. The porin is formed by 7-11 of the porin monomers through a covalent or non-covalent linkage, preferably formed by 9 of the porin monomers.

Further, the porin is formed by 9 porin monomers through a non-covalent linkage, and the porin monomer includes a protein having an amino acid sequence in any one of SEQ ID NO: 1 to SEQ ID NO: 6.

Further, a pore channel diameter of the porin is 0.5-3.0 nm.

In order to implement the above objective, a fourth aspect of the present disclosure provides a kit. The kit includes the porin monomer, or the protein construct, or the porin.

Further, the kit further includes a membrane layer, and the membrane layer includes a lipid layer or an artificial polymer membrane.

Further, the kit further includes at least one of the following: a sequencing buffer, a nuclease, a polymerase, a topoisomerase, a ligase, a helicase, or a cholesterol-linked single-stranded DNA.

In order to implement the above objective, a fifth aspect of the present disclosure provides an isolated DNA molecule. The DNA molecule has a nucleotide sequence encoding the porin monomer, or a nucleotide sequence encoding the protein construct, or a nucleotide sequence encoding the porin.

Further, the DNA molecule has more than 70%, preferably more than 80%, more preferably more than 90%, further preferably more than 95%, and most preferably more than 99% homology to an nucleotide sequence in any one of SEQ ID NO: 7 to SEQ ID NO: 12 and encodes a protein having the same function.

In order to implement the above objective, a sixth aspect of the present disclosure provides a recombinant vector. The recombinant vector contains the DNA molecule.

In order to implement the above objective, a seventh aspect of the present disclosure provides a host cell. The host cell is transformed with the recombinant vector.

In order to implement the above objective, an eighth aspect of the present disclosure provides a nanopore sensor. The nanopore sensor includes a membrane layer; and a porin inserted in the membrane layer and forming a pore channel, where the pore channel generates a current when a voltage is applied across the membrane layer; and the porin includes the above porin.

Further, the membrane layer includes a lipid layer or an artificial polymer membrane; preferably, the lipid layer includes an amphiphilic lipid; preferably, the amphiphilic lipid contains a phospholipid bilayer; preferably, the lipid layer includes a planar membrane layer or a liposome; preferably, the liposome includes a multi-layer liposome or a single-layer liposome; and preferably, the lipid layer includes a phospholipid bilayer consisting of 1,2-diphytanoyl-sn-glycero-3-phosphocholine (DPhPC).

Further, when a voltage is applied across the membrane layer, a biomolecule to be tested passes through the pore channel in the nanopore sensor and undergoes displacement, and the pore channel generates a changing current; preferably, the biomolecule to be tested includes DNA, RNA, or peptide; and preferably, the DNA and/or RNA includes any one or more of the following modified bases: 5-methylcytosine, 6-methyladenine, 7-methylguanine, and pseudouridine.

In order to implement the above objective, a ninth aspect of the present disclosure provides a nanopore sequencing apparatus. The nanopore sequencing apparatus includes the nanopore sensor.

Further, the nanopore sequencing apparatus includes: an electrolytic tank, containing a sequencing buffer; a nanopore sensor, located in the center of the electrolytic tank, and dividing the electrolytic tank and the sequencing buffer into a positive electrode electrolyte region and a negative electrode electrolyte region; and a first electrode and a second electrode, respectively disposed in the positive electrode electrolyte region and the negative electrode electrolyte region, and both connected to a signal processing chip. Preferably, the first electrode and the second electrode are made of metals or composite electrode materials; and preferably, the first electrode and the second electrode are different, and respectively are made of silver and silver chloride; or the first electrode and the second electrode are the same, and are made of gold, platinum, graphene, or titanium nitride.

In order to implement the above objective, a tenth aspect of the present disclosure provides a sequencing method. The sequencing method uses the porin, or the nanopore sensor, or the nanopore sequencing apparatus to detect and analyze an electrical signal generated when a biomolecule to be tested passes through a pore channel of the porin, so as to determine a sequence of the biomolecule to be tested.

Further, the biomolecule to be tested includes modified or unmodified DNA, RNA, or peptide; and preferably, the electrical signal comprises a current.

Further, the biomolecule to be tested is a target nucleotide sequence, and the sequencing method includes the following steps.
(a) the target nucleotide sequence is in contact with the porin and a nucleic acid binding protein such that the nucleic acid binding protein controls a movement speed of the target nucleotide sequence passing through the pore channel of the porin, where the nucleic acid binding protein is selected from any one or more of a nuclease, a polymerase, a topoisomerase, a ligase, a helicase, and a single-strand binding protein; and (b) when applying a voltage across the pore channel, and the target nucleotide sequence moves and passes through the pore channel, an electrical signal passing through the pore channel is measured, where the electrical signals generated by different types of nucleotides passing through the pore channel are different, thereby determining sequence information of the target nucleotide sequence based on the electrical signals.

In order to implement the above objective, an eleventh aspect of the present disclosure provides an application of the porin monomer, or the porin, or the kit, or the DNA molecule, or the recombinant vector, or the host cell, or the nanopore sensor, or the nanopore sequencing apparatus in biological small molecule detection, nucleic acid sequencing, or peptide sequencing.

The present disclosure provides a new porin that may be used for nanopore sequencing and a mutant thereof. The nanopore sensor consisting of the protein or the mutant thereof has a high capture rate to a library, which is advantageous compared to the prior art, thereby improving sequencing throughput and reducing a sample size of the libraries. The porin and the mutant thereof are good in stability, can meet requirements for single-molecule nano sequencing, realize detection of biological small molecules such as nucleotide, amino acid, sugar, vitamin, etc., or may also be used for the sequencing of modified or unmodified DNA, RNA, or peptide.

### Brief Description of the Drawings

The drawings, which form a part of the present disclosure, are used to provide a further understanding of the present disclosure. The exemplary embodiments of the present disclosure and the description thereof are used to explain the present disclosure, but do not constitute improper limitations to the present disclosure. In the drawings:
Fig. 1 is a sideview of a three-dimensional structure of BCP58 obtained through prediction according to Embodiment 1 of the present disclosure.
Fig. 2 is a topview of a three-dimensional structure of BCP58 obtained through prediction according to Embodiment 1 of the present disclosure.
Fig. 3 is a schematic diagram of a prediction structure of a critical amino acid in a BCP58 gating domain and a distance between the critical amino acids according to Embodiment 1 of the present disclosure.
Fig. 4 is an enlarged view of a structure of critical amino acids in a BCP58 gating domain according to Embodiment 1 of the present disclosure.
Fig. 5 is a schematic diagram of charged and polar amino acids of a transmembrane domain toward a membrane in a BCP58 prediction structure according to Embodiment 1 of the present disclosure.
Fig. 6 is a schematic diagram of a critical amino acid at an entrance in a BCP58 prediction structure according to Embodiment 1 of the present disclosure.
Fig. 7 is a schematic diagram of critical amino acids on a pore inner wall and at an entrance in a BCP58 prediction structure according to Embodiment 1 of the present disclosure.
Fig. 8 is an SDS-PAGE diagram obtained through purification of a BCP58 protein according to Embodiment 4 of the present disclosure.
Fig. 9 is a schematic structural diagram of a sequencing library according to Embodiment 5 of the present disclosure, where A is a schematic structural diagram of the sequencing library; B is a schematic structural diagram of the sequencing library bonding to a single-stranded DNA with cholesterol; a: sense strand; b: antisense strand; c: double-stranded target fragment to be tested; d: helicase BCH105; and e: single-stranded DNA with cholesterol.
Fig. 10 shows a specific structure of iSp18 according to Embodiment 5 of the present disclosure.
Figure 11A shows an open-pore current diagram of nanopore protein BCP58 WT in a phospholipid bilayer under different voltages according to Embodiment 6 of the present disclosure.
Figure 11B shows an open-pore current diagram of nanopore protein BCP58 mutant 1 in a phospholipid bilayer under different voltages according to Embodiment 6 of the present disclosure.
Figure 11C shows an open-pore current diagram of nanopore protein BCP58 mutant 2 in a phospholipid bilayer under different voltages according to Embodiment 6 of the present disclosure.
Fig. 12A is a diagram of current changes generated when library DNA passes through a nanopore protein BCP58 WT and local details according to Embodiment 7 of the present disclosure.
Fig. 12B is a diagram of current changes generated when library DNA passes through a nanopore protein BCP58 mutant 1 and local details according to Embodiment 7 of the present disclosure.
Fig. 12C is a diagram of current changes generated when library DNA passes through a nanopore protein BCP58 mutant 2 and local details according to Embodiment 7 of the present disclosure.
Fig. 13A is a current diagram of capture of QC DNA by a nanopore protein BCP58 WT according to Embodiment 8 of the present disclosure.
Fig. 13B is a current diagram of capture of QC DNA by a nanopore protein BCP58 mutant 3 according to Embodiment 8 of the present disclosure.
Fig. 13C is a current diagram of capture of QC DNA by a nanopore protein BCP58 mutant 4 according to Embodiment 8 of the present disclosure.
Fig. 13D is a current diagram of capture of QC DNA by a nanopore protein CSGG-Y51A/F56Q in the prior art according to Embodiment 8 of the present disclosure.

### Detailed Description of the Embodiments

It is to be noted that the embodiments in the present disclosure and the features in the embodiments may be combined with one another without conflict. The present disclosure will be described below in detail with reference to the embodiments.

As mentioned in the Background, porins in the prior art that may be used for single-molecule nanopore sequencing are very few in type, and thus are greatly limited in selectivity. Therefore, in the present disclosure, the inventors have mined a new porin, named BCP58, from a deep-sea metagenome (derived from samples from the 11,000-meter-deep Mariana Trench) by means of gene mining for computer-assisted structure prediction, and the porin is formed by nine monomers of the same type by means of polymerization. The porin has a pore channel structure, and thus may be used as a protein for detection, so as to be applied to detect biological small molecules such as nucleotide, amino acid, sugar, vitamin, etc., or applied to detect nanopore-based DNA, RNA, or peptide. Therefore, a series of protective solutions of the present disclosure are proposed.

A first typical implementation of the present disclosure provides a porin monomer. The porin monomer includes: (a) a protein having an amino acid sequence shown in SEQ ID NO: 1; or (b) a protein mutant, where an amino acid sequence of the protein mutant is subjected to substitution, deletion and/or addition of one or more amino acids at at least one of the following sites in SEQ ID NO: 1: 77, 81, 82, 176, 210, 214, 232, 66, 69, 70, 74, 109, 110, 113, 117, 118, 119, 120, 123, 127, 128, 168, 211, 221, 224, 227, and 229, and the mutant has a function of forming a pore channel structure by means of polymerization; or (c) a protein having at least 70%, 75%, 80%, 85%, 90%, 95%, or 99% homology to the protein in the (a) or the (b), where the mutant has the function of forming the pore channel structure by means of polymerization.

SEQ ID NO: 1:

MKSYMSQISRKRQLMVCLLSLTFAGCVSTPIPPSLESARLTSRTSTYDDLRALPVPKGKIVVSL
YKFNDQTGQYKPSPASSFSTAVTQGATAMLVQALHESGWFIVLEREGLQNLLTERKIIRAAQKKPN
TPVNNADDLPSLLAANVLIEGGVIAYESNVKTGGAGARFLGIGFSEQYRVDQVTVNLRAINVSTGRI
LHNVSTTKTILSKELQSSVYKFVEYKELLELEAGTTTNEPTQMCVRAAIEAAVIHMLVDGLENRSWS
LASESDVSNPVIKRYSEETPKVVIKSAALTSPSDNSLAVMPSATNYYDEIMSGVK.

The porin monomer defined in the (a) can form a porin BCP58 having a pore channel structure by means of polymerization, and can cause biomolecules to be tested to pass through a pore channel one by one when being used for nanopore sequencing, so as to generate a current signal. On the basis of the sequence in the (a), the protein is mutated, for example, mutation is performed in other positions such as mutant sites disclosed in the (b), and after one or more amino acids are substituted and/or deleted and/or added, the pore channel structure and function maintaining the porin can still be obtained. Mutation to the porin monomer may affect the stability, an inner diameter of the pore channel, amino acid residues on an inner wall of the pore channel of the protein and aggregate, thus affecting physical and chemical properties and the through performance of the biomolecule to be tested. However, a conventional operation mode of mutation and a method for obtaining the protein having the nanopore channel structure and functional activity by screening are known to those skilled in the art.

Homology in the specification refers to "homology" between amino acid sequences or nucleotide sequences, that is, the total of ratios of the amino acid residues or nucleotides of the same type in the amino acid sequences or nucleotide sequences. The homology of the amino acid sequences or nucleotide sequences may be determined with comparison programs such as a Basic Local Alignment Search Tool (BLAST), FASTA, etc.

Active sites, active pockets, active mechanisms, protein structures, etc. of proteins having more than 70%, 75%, 80%, 85%, 90%, 95%, 99% (e.g., more than 85%, 86%, 87%, 88%, 89%, 90%, 91%, 92%, 93%, 94%, 95%, 96%, 97%, 98%, 98.5%, 99%, 99.5%, 99.6%, 99.7%, 99.8%, or even more than 99.9%) homology and having the same functions are probably the same as the protein provided the sequence in the (a).

As used herein, the amino acid residues are abbreviated below: Alanine (Ala; A), Asparagine (Asn; N), Aspartic acid (Asp; D), Arginine (Arg; R), Cysteine (Cys; C), Glutamate (Glu; E), Glutamine (Gln; Q), Glycine (Gly; G), Histidine (His; H), Isoleucine (Ile; I), Leucine (Leu; L), Lysine (Lys; K), Methionine (Met; M), Phenylalanine (Phe; F), Proline (Pro; P), Serine (Ser; S), Threonine (Thr; T), Tryptophan (Trp; W), Tyrosine (Tyr; Y), and Valine (Val; V).

Generally, according to rules such as substitution, replacement, etc., amino acids with similar properties have a similar effect when they are substituted for each other. For example, in the protein, conservative amino acid replacements may occur. "Conservative amino acid replacement" includes, but is not limited to:
hydrophobic amino acids (Ala, Cys, Gly, Pro, Met, Val, Ile, and Leu) are substituted by other hydrophobic amino acids;
hydrophobic amino acids with bulky side chains (Phe, Tyr, and Trp) are substituted by other hydrophobic amino acids with bulky side chains;
amino acids with positively charged side chains (Arg, His, and Lys) are substituted by other amino acids with positively charged side chains;
amino acids with polarized and uncharged side chains (Ser, Thr, Asn, and Gln) are substituted by other amino acids with polarized and uncharged side chains.

Those skilled in the art may also make conservative substitutions of the amino acids according to amino acid substitution rules known to those skilled in the art, such as a "blosum62 scoring matrix" in the prior art.

AlphaFold2-Multimer used in the present disclosure is a disclosed artificial intelligence model that can predict protein complex conformation, and can predict a protein three-dimensional structure at a level very close to that observed in a real experiment using devices such as cryo-electron microscopy. A relatively-real protein structure can be obtained to guide the exploration of the protein structure and protein activity.

In a preferred embodiment, in the (b), the types of amino acids substituted are each independently selected as follows: S77 is mutated into S77G, S77A, or S77T; S81 is mutated into S81G, S81A, S81T, S81N, or S81Q; and F82 is mutated into F82G, F82A, F82S, F82T, F82N, or F82Q.

In a three-dimensional structure of a BCP58 protein, three amino acids of an amino acid side chain located in a protein pore channel respectively are S77, S81, and F82. By mutating the three amino acids, the diameter, stability, affinity for passing through molecules, capability of generating an electrical signal, etc. of the pore channel structure of the protein can be changed, thereby obtaining a nanopore protein more suitable for nanopore sequencing, or a nanopore protein having selectivity to a specific molecule to be tested.

In a preferred embodiment, in the (b), the types of amino acids substituted are each independently selected as follows: E176 is mutated into E176A, E176G, E176V, E176L, E176I, E176Y, E176F, or E176W; K210 is mutated into K210A, K210G, K210V, K210L, K210I, K210Y, K210F, or K210W; S214 is mutated into S214A, S214G, S214V, S214L, S214I, S214Y, S214F, or S214W; and T232 is mutated into T232A, T232G, T232V, T232L, T232I, T232Y, T232F, or T232W.

The above four amino acid sites are located on a side of a porin transmembrane domain toward a membrane, and mutation to the amino acid sites may improve the performance of nanopore protein membrane insertion.

In a preferred embodiment, in the (b), the types of amino acids substituted are each independently selected as follows: K66 is mutated into K66N, K66A, K66G, K66S, K66T, or K66Q; D69 is mutated into D69K, D69R, D69N, D69A, D69G, D69S, D69T, or D69Q; Q70 is mutated into Q70K, Q70R, Q70D, Q70E, Q70N, Q70A, Q70G, Q70S, Q70T, or Q70Q; Y74 is mutated into Y74N, Y74A, Y74G, Y74S, Y74T, or Y74Q; R109 is mutated into R109N, R109A, R109G, R109S, R109T, or R109Q; E110 is mutated into E110K, E110R, E110N, E110A, E110G, E110S, E110T, or E110Q; Q113 is mutated into Q113K, Q113R, Q113N, Q113A, Q113G, Q113S, or Q113T; T117 is mutated into T117K, T117R, T117N, T117A, T117G, T117S, or T117Q; E118 is mutated into E118K, E118R, E118N, E118A, E118G, E118S, E118T, or E118Q; R119 is mutated into R119N, R119A, R119G, R119S, R119T, or R119Q; K120 is mutated into K120N, K120A, K120G, K120S, K120T, or K120Q; R123 is mutated into R123N, R123A, R123G, R123S, R123T, or R123Q; K127 is mutated into K127N, K127A, K127G, K127S, K127T, or K127Q; K128 is mutated into K128N, K128A, K128G, K128S, K128T, or K128Q; R168 is mutated into R168N, R168Q, R168S, R168T, R168A, or R168G; E211 is mutated into E211N, E211Q, E211S, E211T, E211A, or E211G; E221 is mutated into E221N, E221Q, E221S, E221T, E221A, or E221G; E224 is mutated into E224N, E224Q, E224S, E224T, E224A, or E224G; E227 is mutated into E227N, E227Q, E227S, E227T, E227A, or E227G; and E229 is mutated into E229N, E229Q, E229S, E229T, E229A, or E229G.

Most of the amino acid sites are at an entrance, inner pore wall, and exit of the porin, and is of important significance on the capture of a library and the smooth and unobstructed passing of library DNA through the porin. Since nucleic acids are negatively charged, the library capture capability of the porin may be adjusted by increasing or reducing the amount of positive charge at the entrance.

The above mutant sites mainly include three types of sites: the mutant sites in a gating domain (sensor domain), the mutant sites at the entrance of the porin, and the mutant sites in the porin transmembrane domain. The mutant sites in the sensor domain mainly determine an open-pore situation of the porin, thereby directly determining an open-pore current. Since a nucleic acid to be tested (a target nucleotide sequence) is negatively charged, by adjusting the amino acids of the mutant sites at the entrance of the porin, including but not limited to, mutating non-charged or negatively charged amino acids into positively charged amino acids, or mutating the positively charged amino acids into other types of amino acids, a capture rate of the library can be adjusted. The mutation in the porin transmembrane domain can improve the membrane insertion stability of the porin on a lipid or polymer membrane. In addition, the charged amino acids located on the inner wall or at the exit of the pore channel structure of the porin can also affect the perforation of a sample to be tested.

In the above mutant sites, the S77, the S81, and the F82 are located in the sensor domain. The K66, the D69, the Q70, the Y74, the R109, the E110, the Q113, the T117, the E118, the R119, the K120, the R123, the K127, and the K128 are located at the entrance of the porin, and mutation to charged properties may play an important role in adjusting the capture rate of the library and sequencing noise. The E176, the K210, the S214, and the T232 are located on an outer wall of the porin transmembrane domain, and the stability of porin membrane insertion can be improved by mutating the above into hydrophobic amino acids. The R168, the E211, the E227, and the E229 are located on an inner wall of the pore channel structure, which are charged amino acids on the inner wall of the porin, and mutation to the charge of the above can promote the molecule to be tested such as DNA to be tested to smoothly pass through the porin. The E221 and the E224 are located in a loop region at the exit of the pore channel structure, and mutation to the above can promote a sequenced nucleic acid chain to separate from the porin.

In a preferred embodiment, in the (b), the types of amino acids substituted are each independently selected as follows: S77A, F82Q, S81N, F82A, K120N, K127S, and E176I.

In a preferred embodiment, the porin monomer includes a protein having an amino acid sequence in any one of SEQ ID NO: 2 to SEQ ID NO: 6.
SEQ ID NO: 2: 
MKSYMSQISRKRQLMVCLLSLTFAGCVSTPIPPSLESARLTSRTSTYDDLRALPVPKGKIVVSL
YKFNDQTGQYKPAPASSQSTAVTQGATAMLVQALHESGWFIVLEREGLQNLLTERKIIRAAQKKPN
TPVNNADDLPSLLAANVLIEGGVIAYESNVKTGGAGARFLGIGFSEQYRVDQVTVNLRAINVSTGRI
LHNVSTTKTILSKELQSSVYKFVEYKELLELEAGTTTNEPTQMCVRAAIEAAVIHMLVDGLENRSWS
 LASESDVSNPVIKRYSEETPKVVIKSAALTSPSDNSLAVMPSATNYYDEIMSGVK.
SEQ ID NO: 3: 
MKSYMSQISRKRQLMVCLLSLTFAGCVSTPIPPSLESARLTSRTSTYDDLRALPVPKGKIVVSL
YKFNDQTGQYKPSPASNASTAVTQGATAMLVQALHESGWFIVLEREGLQNLLTERKIIRAAQKKPN
TPVNNADDLPSLLAANVLIEGGVIAYESNVKTGGAGARFLGIGFSEQYRVDQVTVNLRAINVSTGRI
LHNVSTTKTILSKELQSSVYKFVEYKELLELEAGTTTNEPTQMCVRAAIEAAVIHMLVDGLENRSWS
LASESDVSNPVIKRYSEETPKVVIKSAALTSPSDNSLAVMPSATNYYDEIMSGVK.
SEQ ID NO: 4: 
MKSYMSQISRKRQLMVCLLSLTFAGCVSTPIPPSLESARLTSRTSTYDDLRALPVPKGKIVVSL
YKFNDQTGQYKPSPASNASTAVTQGATAMLVQALHESGWFIVLEREGLQNLLTERNIIRAAQKKPN
TPVNNADDLPSLLAANVLIEGGVIAYESNVKTGGAGARFLGIGFSEQYRVDQVTVNLRAINVSTGRI
LHNVSTTKTILSKELQSSVYKFVEYKELLELEAGTTTNEPTQMCVRAAIEAAVIHMLVDGLENRSWS
LASESDVSNPVIKRYSEETPKVVIKSAALTSPSDNSLAVMPSATNYYDEIMSGVK.
SEQ ID NO: 5: 
MKSYMSQISRKRQLMVCLLSLTFAGCVSTPIPPSLESARLTSRTSTYDDLRALPVPKGKIVVSL
YKFNDQTGQYKPSPASNASTAVTQGATAMLVQALHESGWFIVLEREGLQNLLTERKIIRAAQSKPN
TPVNNADDLPSLLAANVLIEGGVIAYESNVKTGGAGARFLGIGFSEQYRVDQVTVNLRAINVSTGRI
LHNVSTTKTILSKELQSSVYKFVEYKELLELEAGTTTNEPTQMCVRAAIEAAVIHMLVDGLENRSWS
LASESDVSNPVIKRYSEETPKVVIKSAALTSPSDNSLAVMPSATNYYDEIMSGVK.
SEQ ID NO: 6:

MKSYMSQISRKRQLMVCLLSLTFAGCVSTPIPPSLESARLTSRTSTYDDLRALPVPKGKIVVSL
YKFNDQTGQYKPSPASSFSTAVTQGATAMLVQALHESGWFIVLEREGLQNLLTERKIIRAAQKKPN
TPVNNADDLPSLLAANVLIEGGVIAYESNVKTGGAGARFLGIGFSIQYRVDQVTVNLRAINVSTGRIL
HNVSTTKTILSKELQSSVYKFVEYKELLELEAGTTTNEPTQMCVRAAIEAAVIHMLVDGLENRSWSL
ASESDVSNPVIKRYSEETPKVVIKSAALTSPSDNSLAVMPSATNYYDEIMSGVK.

A second typical implementation of the present disclosure provides a protein construct. The protein construct is formed by 2 or more porin monomers through a covalent or non-covalent linkage.

A third typical implementation of the present disclosure provides a porin. The porin is formed by 7-11 of the porin monomers through a covalent or non-covalent linkage, preferably formed by 9 of the porin monomers.

The porin monomers can be spontaneously polymerized together through forces such as a hydrogen bond, an ionic bond, a hydrophobic interaction, etc., so as to form the porin by means of polymerization. Therefore, the porin monomers obtained through expression and purification are present in the form of polymers, in particular, nonamers under non-denaturing conditions, and denaturing the protein results in the formation of porin monomers.

In a preferred embodiment, the porin is formed by 9 porin monomers through a non-covalent linkage, and the porin monomer includes a protein having an amino acid sequence in any one of SEQ ID NO: 1 to SEQ ID NO: 6.

In a preferred embodiment, a pore channel diameter of the porin is 0.5-3.0 nm.

Within a certain range, if the pore channel diameter of the nanopore protein is smaller, the accuracy of the nanopore protein for sequencing is higher. If the pore channel diameter of the nanopore protein is too large (there may be more than one molecule passing through the pore channel at a time), it is difficult to meet requirements for single-molecule sequencing, and when a biomolecule to be tested passes through the too large pore channel, a current signal generated may be missed or generate errors, resulting in low accuracy of sequencing. During single-molecule sequencing, an accurate sequencing result is obtained by means of performing sequencing on the same molecule for a plurality of times. Therefore, if the accuracy of sequencing is higher, the number of times and time required for sequencing are longer. With the nanopore protein with high sequencing accuracy for sequencing, the time for sequencing can be greatly shortened, the costs can be reduced, and this advantage is particularly evident in high-throughput sequencing.

In a fourth typical implementation of the present disclosure provides a kit. The kit includes the porin monomer, or the protein construct, or the porin.

In order to further improving operational convenience, in a preferred embodiment, the kit further includes a membrane layer, and the membrane layer includes a lipid layer or an artificial polymer membrane.

Preferably, the lipid layer includes an amphiphilic lipid; preferably, the amphiphilic lipid contains a phospholipid bilayer; preferably, the lipid layer includes a planar membrane layer or a liposome; preferably, the liposome includes a multi-layer liposome or a single-layer liposome; and preferably, the lipid layer includes a phospholipid bilayer consisting of DPhPC.

The artificial polymer membrane includes, but is not limited to, one or more of polysiloxane, polyolefin, perfluoropolyether, perfluoroalkyl polyether, polystyrene, polypropylene oxide, poly(vinyl acetate), poly(butylene oxide), polyisoprene, polybutadiene, polyvinyl chloride, polyalkylacrylate, polyalkyl methyl methacrylate, polyacrylonitrile, polypropylene, PTHF, polymethacrylate, polyacrylate, polysulfone, polyvinylether, poly(propylene oxide) and its copolymers, substituted C1-C6 alkyl acrylate and methacrylate, acrylamide, methacrylamide, (C1-C6 alkyl) acrylamide and methacrylamide, N,N-dialkyl-acrylamide, ethoxy acrylate and methacrylate, poly(ethylene glycol) (n) monomethacrylate and polyethylene glycol methyl ether methacrylate, hydroxyl-substituted (C1-C6 alkyl) acrylamide and methacrylamide, hydroxyl-substituted C1-C6 alkyl vinyl ether, sodium vinyl sulfonate, sodium styryl sulfonate, 2-acrylamide-2-methylpropanesulfonic acid, N-vinylpyrrole, N-vinyl-2-pyrrolidone, 2-vinyloxazoline, 2-vinyl-4,4'-dialkyl oxazolinyl-5-one, 2,4-vinylpyridine, vinylated unsaturated carboxylic acid totally having 3-5 carbon atoms, amino (C1 to C6 alkyl)-, mono(C1-C6 alkylamino)(C1-C6 alkyl)- and bis(C1-C6 alkylamino)(C1-C6 alkyl)-acrylate and methacrylate, allyl alcohol, 2-hydroxypropyl 3-trimethylammonium methacrylate chloride, dimethyl aminoethyl methacrylate (DMAEMA), dimethyl aminoethyl methacrylamide, glycerol methacrylate, N-(1,1-dimethyl-3-oxobutyl)acrylamide, cyclic imino ether, vinyl ether, cyclic ether containing epoxy derivative, cyclic unsaturated ether, N-substituted aziridine, β-lactone and β-lactam, ketene acetal, vinyl acetal, and phosphorane.

In a preferred embodiment, the kit further includes at least one of the following: a sequencing buffer, a nuclease, a polymerase, a topoisomerase, a ligase, a helicase, or a cholesterol-linked single-stranded DNA.

With the nanopore protein, membrane layer, and sequencing buffer in the kit, one or more nanopore proteins can insert into the membrane layer in the sequencing buffer, so as to form a nanopore sensor. The nanopore experiment buffer can provide a neutral environment maintaining the stability of the nanopore protein and the membrane layer, and metal ions contained therein cause the nanopore experiment buffer to have good conductivity. There are various options for the membrane layer. The nanopore protein can be inserted in the planar membrane layer or spherical liposome, or in the lipid layer formed by different components, so as to form the nanopore sensor.

The cholesterol carried on the molecule to be tested such as single-stranded DNA may bind to the lipid layer or the artificial polymer membrane, facilitating the capture of a sequencing library by the nanopore, thereby reducing an input amount of the sequencing libraries. The cholesterol in the kit may first bind to the molecule to be tested during actual use, and then be added to a space in which the nanopore sensor is located for sequencing.

A fifth typical implementation of the present disclosure provides an isolated DNA molecule. The DNA molecule has a nucleotide sequence encoding the porin monomer, or a nucleotide sequence encoding the protein construct, or a nucleotide sequence encoding the porin.

In a preferred embodiment, the DNA molecule has more than 70%, preferably more than 80%, more preferably more than 90%, further preferably more than 95%, and most preferably more than 99% (which may be, for example, more than 85%, 86%, 87%, 88%, 89%, 90%, 91%, 92%, 93%, 94%, 95%, 96%, 97%, 98%, 98.5%, 99%, 99.5%, 99.6%, 99.7%, 99.8%, or even more than 99.9%) homology to an nucleotide sequence in any one of SEQ ID NO: 7 to SEQ ID NO: 12 and encodes a protein having the same function.
SEQ ID NO: 7: 
atgaaaagctatatgagccagattagccgtaaacgccagctgatggtttgtctgctgagcctgacctttgcaggttgtgttagcacccc
gattccgccgagtctggaaagtgcacgtttaacaagtcgtaccagcacctatgatgatctgcgtgcactgccggttccgaaaggtaaaattg
ttgttagcctgtataagttcaacgaccagaccggtcagtataagccgagcccggcaagtagctttagcaccgcagttacccagggtgcaa
ccgcaatgctggttcaggcactgcatgaaagcggttggtttattgttctggaacgtgaaggtctgcagaatctgctgaccgaacgtaaaatta
ttcgcgcagcacagaaaaaaccgaataccccggttaataatgccgatgatctgccgagcctgctggcagcaaatgttctgattgaaggtg
gtgttattgcctatgaaagcaatgttaaaaccggcggcgcaggtgcacgttttctgggtattggttttagcgaacagtatcgggttgatcaggtt
accgttaatctgcgtgcaattaatgttagcaccggtcgtattctgcataatgttagcaccaccaaaaccattctgagcaaagaactgcagag
cagcgtttataaatttgttgagtataaggagctgctggagctggaggcaggtaccaccacaaatgaaccgacccagatgtgtgttcgtgca
gcaattgaagcagcagttattcacatgctggttgacggtctggaaaatcgtagctggagcctggcaagcgaaagcgatgttagcaatccg
gttattaaacgttatagcgaggaaaccccgaaagttgttattaaaagcgcagcactgaccagcccgagcgataatagcctggcagttatg
ccgagcgcaaccaattattatgatgaaattatgagcggcgtgaagtaa.
SEQ ID NO: 8: 
atgaaaagctatatgagccagattagccgtaaacgccagctgatggtttgtctgctgagcctgacctttgcaggttgtgttagcacccc
gattccgccgagtctggaaagtgcacgtttaacaagtcgtaccagcacctatgatgatctgcgtgcactgccggttccgaaaggtaaaattg
ttgttagcctgtataagttcaacgaccagaccggtcagtataagccggcaccggcaagtagccagagcaccgcagttacccagggtgca
accgcaatgctggttcaggcactgcatgaaagcggttggtttattgttctggaacgtgaaggtctgcagaatctgctgaccgaacgtaaaatt
attcgcgcagcacagaaaaaaccgaataccccggttaataatgccgatgatctgccgagcctgctggcagcaaatgttctgattgaaggt
ggtgttattgcctatgaaagcaatgttaaaaccggcggcgcaggtgcacgttttctgggtattggttttagcgaacagtatcgggttgatcagg
ttaccgttaatctgcgtgcaattaatgttagcaccggtcgtattctgcataatgttagcaccaccaaaaccattctgagcaaagaactgcaga
gcagcgtttataaatttgttgagtataaggagctgctggagctggaggcaggtaccaccacaaatgaaccgacccagatgtgtgttcgtgc
agcaattgaagcagcagttattcacatgctggttgacggtctggaaaatcgtagctggagcctggcaagcgaaagcgatgttagcaatcc
ggttattaaacgttatagcgaggaaaccccgaaagttgttattaaaagcgcagcactgaccagcccgagcgataatagcctggcagttat
gccgagcgcaaccaattattatgatgaaattatgagcggcgtgaagtaa.
SEQ ID NO: 9: 
atgaaaagctatatgagccagattagccgtaaacgccagctgatggtttgtctgctgagcctgacctttgcaggttgtgttagcacccc
gattccgccgagtctggaaagtgcacgtttaacaagtcgtaccagcacctatgatgatctgcgtgcactgccggttccgaaaggtaaaattg
ttgttagcctgtataagttcaacgaccagaccggtcagtataagccgagcccggcaagtaatgcaagcaccgcagttacccagggtgca
accgcaatgctggttcaggcactgcatgaaagcggttggtttattgttctggaacgtgaaggtctgcagaatctgctgaccgaacgtaaaatt
attcgcgcagcacagaaaaaaccgaataccccggttaataatgccgatgatctgccgagcctgctggcagcaaatgttctgattgaaggt
ggtgttattgcctatgaaagcaatgttaaaaccggcggcgcaggtgcacgttttctgggtattggttttagcgaacagtatcgggttgatcagg
ttaccgttaatctgcgtgcaattaatgttagcaccggtcgtattctgcataatgttagcaccaccaaaaccattctgagcaaagaactgcaga
gcagcgtttataaatttgttgagtataaggagctgctggagctggaggcaggtaccaccacaaatgaaccgacccagatgtgtgttcgtgc
agcaattgaagcagcagttattcacatgctggttgacggtctggaaaatcgtagctggagcctggcaagcgaaagcgatgttagcaatcc
ggttattaaacgttatagcgaggaaaccccgaaagttgttattaaaagcgcagcactgaccagcccgagcgataatagcctggcagttat
gccgagcgcaaccaattattatgatgaaattatgagcggcgtgaagtaa.
SEQ ID NO: 10: 
atgaaaagctatatgagccagattagccgtaaacgccagctgatggtttgtctgctgagcctgacctttgcaggttgtgttagcacccc
gattccgccgagtctggaaagtgcacgtttaacaagtcgtaccagcacctatgatgatctgcgtgcactgccggttccgaaaggtaaaattg
ttgttagcctgtataagttcaacgaccagaccggtcagtataagccgagcccggcaagtaatgcaagcaccgcagttacccagggtgca
accgcaatgctggttcaggcactgcatgaaagcggttggtttattgttctggaacgtgaaggtctgcagaatctgctgaccgaacgtaatatt
attcgcgcagcacagaaaaaaccgaataccccggttaataatgccgatgatctgccgagcctgctggcagcaaatgttctgattgaaggt
ggtgttattgcctatgaaagcaatgttaaaaccggcggcgcaggtgcacgttttctgggtattggttttagcgaacagtatcgggttgatcagg
ttaccgttaatctgcgtgcaattaatgttagcaccggtcgtattctgcataatgttagcaccaccaaaaccattctgagcaaagaactgcaga
gcagcgtttataaatttgttgagtataaggagctgctggagctggaggcaggtaccaccacaaatgaaccgacccagatgtgtgttcgtgc
agcaattgaagcagcagttattcacatgctggttgacggtctggaaaatcgtagctggagcctggcaagcgaaagcgatgttagcaatcc
ggttattaaacgttatagcgaggaaaccccgaaagttgttattaaaagcgcagcactgaccagcccgagcgataatagcctggcagttat
gccgagcgcaaccaattattatgatgaaattatgagcggcgtgaagtaa.
SEQ ID NO: 11: 
atgaaaagctatatgagccagattagccgtaaacgccagctgatggtttgtctgctgagcctgacctttgcaggttgtgttagcacccc
gattccgccgagtctggaaagtgcacgtttaacaagtcgtaccagcacctatgatgatctgcgtgcactgccggttccgaaaggtaaaattg
ttgttagcctgtataagttcaacgaccagaccggtcagtataagccgagcccggcaagtaatgcaagcaccgcagttacccagggtgca
accgcaatgctggttcaggcactgcatgaaagcggttggtttattgttctggaacgtgaaggtctgcagaatctgctgaccgaacgtaaaatt
attcgcgcagcacagagcaaaccgaataccccggttaataatgccgatgatctgccgagcctgctggcagcaaatgttctgattgaaggt
ggtgttattgcctatgaaagcaatgttaaaaccggcggcgcaggtgcacgttttctgggtattggttttagcgaacagtatcgggttgatcagg
ttaccgttaatctgcgtgcaattaatgttagcaccggtcgtattctgcataatgttagcaccaccaaaaccattctgagcaaagaactgcaga
gcagcgtttataaatttgttgagtataaggagctgctggagctggaggcaggtaccaccacaaatgaaccgacccagatgtgtgttcgtgc
 
agcaattgaagcagcagttattcacatgctggttgacggtctggaaaatcgtagctggagcctggcaagcgaaagcgatgttagcaatcc
ggttattaaacgttatagcgaggaaaccccgaaagttgttattaaaagcgcagcactgaccagcccgagcgataatagcctggcagttat
gccgagcgcaaccaattattatgatgaaattatgagcggcgtgaagtaa.
SEQ ID NO: 12:

atgaaaagctatatgagccagattagccgtaaacgccagctgatggtttgtctgctgagcctgacctttgcaggttgtgttagcacccc
gattccgccgagtctggaaagtgcacgtttaacaagtcgtaccagcacctatgatgatctgcgtgcactgccggttccgaaaggtaaaattg
ttgttagcctgtataagttcaacgaccagaccggtcagtataagccgagcccggcaagtagctttagcaccgcagttacccagggtgcaa
ccgcaatgctggttcaggcactgcatgaaagcggttggtttattgttctggaacgtgaaggtctgcagaatctgctgaccgaacgtaaaatta
ttcgcgcagcacagaaaaaaccgaataccccggttaataatgccgatgatctgccgagcctgctggcagcaaatgttctgattgaaggtg
gtgttattgcctatgaaagcaatgttaaaaccggcggcgcaggtgcacgttttctgggtattggttttagcattcagtatcgggttgatcaggtta
ccgttaatctgcgtgcaattaatgttagcaccggtcgtattctgcataatgttagcaccaccaaaaccattctgagcaaagaactgcagagc
agcgtttataaatttgttgagtataaggagctgctggagctggaggcaggtaccaccacaaatgaaccgacccagatgtgtgttcgtgcag
caattgaagcagcagttattcacatgctggttgacggtctggaaaatcgtagctggagcctggcaagcgaaagcgatgttagcaatccggt
tattaaacgttatagcgaggaaaccccgaaagttgttattaaaagcgcagcactgaccagcccgagcgataatagcctggcagttatgcc
gagcgcaaccaattattatgatgaaattatgagcggcgtgaagtaa.

The DNA molecule can encode the nanopore protein having the above structure and function of the present disclosure. The nucleotides are mutated on the basis of the sequence in the (a), hybridized with the DNA molecule defined in the (a) under a stringent condition, and without frameshift mutations. If the mutation occurs in the nucleotides on the pore channel of the encoded nanopore protein, the nanopore protein with changed pore channel may be encoded, affecting the pore diameter of the nanopore protein and the properties of amino acid residues on the inner wall of the pore channel; and if the mutation occurs in the nucleotides at a non-pore channel portion of the encoded protein, the properties of the encoded protein such as a folding pattern, a three-dimensional structure, etc., may be affected, thus affecting the physical and chemical properties and stability of the protein. The nucleotide sequence for hybridization of the DNA molecule defined in the (b) under the stringent condition includes a nucleotide sequence having 80%, 85%, 90%, 95%, 98%, 99%, 99.9%, or 100% complementary to the DNA molecule defined in the (a).

The term "isolated" in the present disclosure means "artificially" changed from its natural state, that is, if it occurs in nature, it changes and/or separates it from its original environment. For example, a polynucleotide or polypeptide that is naturally present in a living organism is not "isolated", however, the same polynucleotide or polypeptide that is isolated from a coexisting material in its natural state is "isolated" (as the term used herein).

A sixth typical implementation of the present disclosure provides a recombinant vector. The recombinant vector contains the DNA molecule.

A nanopore protein expressed gene is obtained by inserting the DNA molecule on the recombinant vector, and the nanopore protein expressed genes are massively replicated with a function of the recombinant vector capable of massive self-replication. "Recombinant" here means genetically engineered DNA prepared by transplanting or splicing genes from one species into cells of a host organism of a different species. Such DNA becomes a portion of a host gene structure and is replicated.

A seventh typical implementation of the present disclosure provides a host cell. The recombinant vector is transformed in the host cell.

The recombinant vector is transformed in the host cell, and a large number of nanopore proteins can be generated with the host cell to replicate, transcribe, and translate a nanopore protein expressed gene on the recombinant vector. The host cell includes common host cells such as *Escherichia coli,* yeast, mammalian cells, insect cells, etc. The nanopore protein is folded with the host cell to form it into a correct three-dimensional structure, so as to obtain the nanopore protein with a normal structure and function.

An eighth typical implementation of the present disclosure provides a nanopore sensor. The nanopore sensor includes a membrane layer; and a porin inserted in the membrane layer and forming a pore channel. The pore channel generates a current when a voltage is applied across the membrane layer; and the porin includes the above porin.

The nanopore sensor in the present disclosure particularly refers to a membrane layer with a nanopore protein inserted. In the nanopore sensor, by inserting the nanopore protein with a pore channel into the membrane layer, the orientation of the pore channel of the nanopore protein can be fixed; when an electric field force is applied across the membrane layer, a diameter of the pore channel is perpendicular to a direction of the electric field force; and ions on two sides of the membrane layer pass through the pore channel of the porin under the action of the electric field force, so as to generate a current.

In a preferred embodiment, the membrane layer includes a lipid layer or an artificial polymer membrane; preferably, the lipid layer includes an amphiphilic lipid; preferably, the amphiphilic lipid contains a phospholipid bilayer; preferably, the lipid layer includes a planar membrane layer or a liposome; preferably, the liposome includes a multi-layer liposome or a single-layer liposome; and preferably, the lipid layer includes a phospholipid bilayer consisting of DPhPC.

There are various options for the lipid layer. The nanopore protein can be inserted in the planar membrane layer or spherical liposome, or in the lipid layer formed by different components, so as to form the nanopore sensor.

In a preferred embodiment, when a voltage is applied across the membrane layer, a biomolecule to be tested passes through the pore channel in the nanopore sensor and undergoes displacement, and the pore channel generates a changing current; preferably, the biomolecule to be tested includes DNA, RNA, or peptide; and preferably, the DNA and/or RNA includes any one or more of the following modified bases: 5-methylcytosine (5mC), 6-methyladenine (m6A), 7-methylguanine (m7G), and pseudouridine (Ψ).

When the electric field force is applied across the membrane layer, the biomolecule to be tested passes through the nanopore protein via the pore channel under the action of the electric field force. The biomolecule to be tested includes biomacromolecules carrying biological genetic information such as DNA, RNA, peptide or protein, etc. The biomolecule to be tested may carry a group molecule for modification. The group molecule includes, but is not limited to, cholesterol, polyethylene glycol with different degrees of polymerization, biotin, or a fluorescent group molecule.

A ninth typical implementation of the present disclosure provides a nanopore sequencing apparatus. The nanopore sequencing apparatus includes the nanopore sensor.

In a preferred embodiment, the nanopore sequencing apparatus includes: an electrolytic tank, containing a sequencing buffer; a nanopore sensor, located in the center of the electrolytic tank, and dividing the electrolytic tank and the sequencing buffer into a positive electrode electrolyte region and a negative electrode electrolyte region; and a first electrode and a second electrode, respectively disposed in the positive electrode electrolyte region and the negative electrode electrolyte region, and both connected to a signal processing chip. Preferably, the first electrode and the second electrode are made of metals or composite electrode materials; and preferably, the first electrode and the second electrode are different, and respectively are made of silver and silver chloride; or the first electrode and the second electrode are the same, and are made of gold, platinum, graphene, or titanium nitride.

The nanopore sequencing apparatus includes the electrolytic tank containing electrolyte, the nanopore sensor, the first electrode, and the second electrode. The nanopore sensor is put in the center of the electrolytic tank containing the electrolyte, the electrolytic tank is divided into the positive electrode electrolyte region and the negative electrode electrolyte region; the 2 regions are respectively provided with 2 electrodes; and an electric field applied to the nanopore sensor is formed with the 2 electrodes. A biomolecule to be tested generates a current amplitude by passing through a nanopore protein on a membrane. The current amplitude is received and transmitted to a signal processing chip connected to the electrodes. According to different current amplitudes, the signal processing chip, that is, the nanopore sequencing apparatus including the signal processing chip can perform data analysis and determination on a sequence of the biomolecule to be tested.

A tenth typical implementation of the present disclosure provides a sequencing method. The sequencing method uses the porin, or the nanopore sensor, or the nanopore sequencing apparatus to detect and analyze an electrical signal generated when a biomolecule to be tested passes through a pore channel of the porin, so as to determine a sequence of the biomolecule to be tested.

In a preferred embodiment, the biomolecule to be tested includes modified or unmodified DNA, RNA, or peptide; and preferably, the electrical signal comprises a current.

In a preferred embodiment, the biomolecule to be tested is a target nucleotide sequence. The sequencing method includes: (a) a target nucleotide sequence is in contact with the porin and a nucleic acid binding protein such that the nucleic acid binding protein controls a movement speed of the target nucleotide sequence passing through the pore channel of the porin, where the nucleic acid binding protein is selected from any one or more of a nuclease, a polymerase, a topoisomerase, a ligase, a helicase, and a single-strand binding protein; and (b) when applying a voltage across the pore channel, and when the target nucleotide sequence moves and passes through the pore channel, an electrical signal passing through the pore channel is measured, where the electrical signals generated by different types of nucleotides passing through the pore channel are different, thereby determining sequence information of the target nucleotide sequence based on the electrical signals.

An eleventh typical implementation of the present disclosure provides an application of the porin monomer, or the porin, or the kit, or the DNA molecule, or the recombinant vector, or the host cell, or the nanopore sensor, or the nanopore sequencing apparatus in biological small molecule detection, nucleic acid sequencing, or peptide sequencing.

An extremely long read length may be achieved based on one of the major advantages of current nanopore sequencing over traditional sequencing: accuracy is not affected by the accumulation of errors. Therefore, the problems of short traditional sequencing and an unavoidable gap during the assembly of a sequencing fragment may be made up, and whether the deletion, duplication, inversion, or translocation of a long fragment occurs in a chromosome and a full length of a transcriptome typically several kbs in length is covered are determined, such that brand new solutions are provided for scientific research such as genome assembly, structure variation, alternative splicing, etc.

Since PCR amplification is not required for nanopore sequencing, original base modification information on a nucleic acid molecule to be tested may be reserved, thereby directly learning the types, sites, and abundances of modified bases through one-time sequencing. Therefore, several nucleic acid molecules carrying DNA/RNA modified bases can also be detected by the nanopore protein of the present disclosure: including 5-methylcytosine (5mC), 6-methyladenine (m6A), 7-methylguanine (m7G), pseudouridine (Ψ), etc. By performing specific model training and algorithm development on various modified bases, nanopore sequencing may complete identification and positioning of more modified bases, thereby constructing a more complete genome/transcriptome modification chart.

Furthermore, considering from the point of view of clinical application, nanopore sequencing has the characteristics of being long in read length, high in convenience, fast in sequencing speed, and real-time in reading, thus is suitable for being used in major epidemic monitoring and rapid pathogen detection (e.g., actions against large-scale epidemics such as Zika viruses, Ebola viruses, Dengue viruses, Coronavirus, etc.), and has timeliness. In addition to viruses, nanopore sequencing may also be used for rapid detection of other pathogens such as bacteria, fungi, etc.

Based on commonality of composition of proteins and nucleic acid molecules, a nanopore sequencing platform also has huge application potential in the field of protein sequencing. For example, based on what has been explored so far, it is known that, with a protein unfolding enzyme as a speed control tool, a protein characteristic signal is successfully observed, and preliminary identification of protein types and modification states are realized, thereby verifying the possibility of nanopore protein sequencing. In the future development, by further optimizing a speed control system and developing an adaptive nanopore protein and signal analysis algorithm, fingerprint recognition and even sequence identification on the protein at a single-molecule level may be realized ultimately.

In addition to applications in sequencing, the nanopore platform may also be used as a basic detection platform, which completes metabolomics detection of various small molecules and large molecules in combination with sensing means. In combination with genomics, proteomics, and metabolomics, the nanopore platform may be ultimately developed into a universal measurement platform meeting whole-omics analysis requirements, thereby providing a powerful research tool for a deeper understanding of the laws of life and disease occurrence mechanisms.

The beneficial effects of the present disclosure are further described in detail below with reference to specific embodiments. However, it will be understood by those skilled in the art that the following embodiments are intended to illustrate the present disclosure only and should not be considered as limiting the scope of the present disclosure. The reagents or instruments used of which the manufacturers are not indicated are conventional products that are commercially available. The experimental methods used are conventional if not otherwise stated.

### Embodiment 1 Prediction structure of AlphaFold2-Multimer of wild-type BCP58

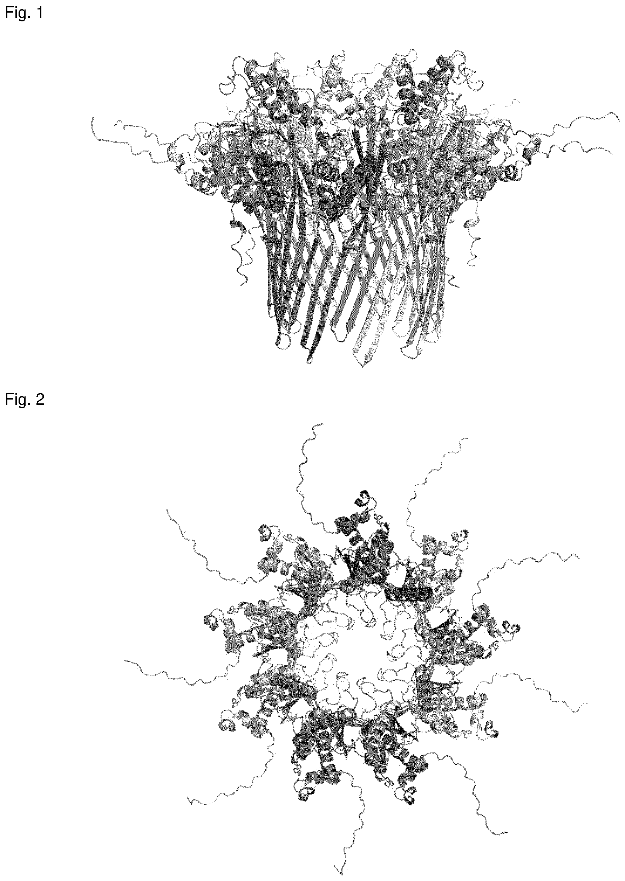

Nine porin monomers (SEQ ID NO: 1) were non-covalently polymerized into a nonamer, so as to obtain a porin BCP58, and structure prediction was performed on the BCP58 with AlphaFold2-Multimer. Prediction results were shown in Fig. 1, Fig. 2, Fig. 3, Fig. 4, Fig. 5, Fig. 6, and Fig. 7. Fig. 1 was a sideview of a prediction structure of the BCP58; and Fig. 2 was a topview of the prediction structure of the BCP58.

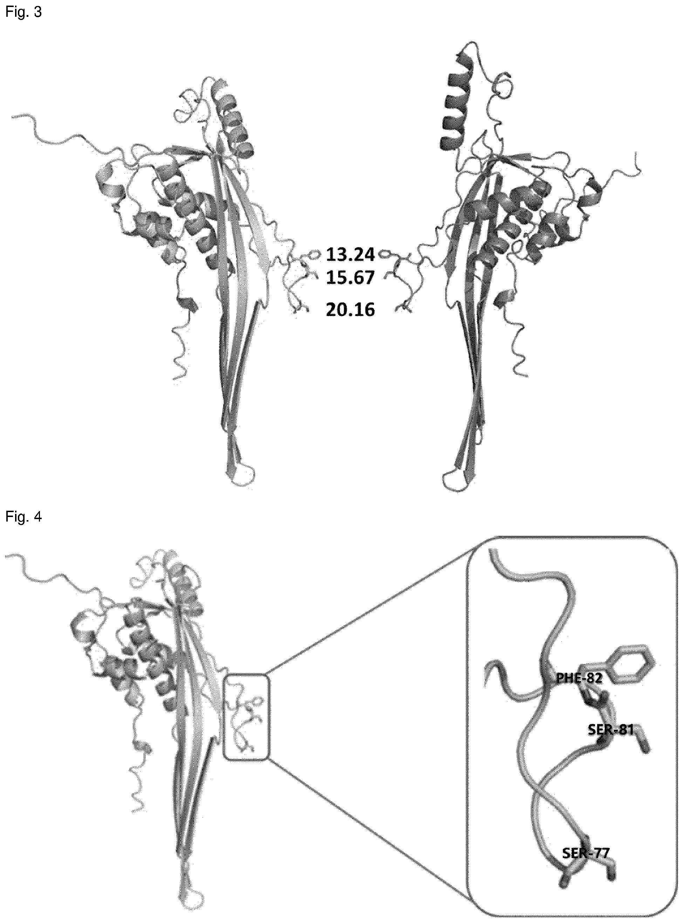

Since compositions of amino acids in a sensor domain were decisive for a current signal, mutants of BCP52 were first tried as mutations for its sensor domain. Fig. 3 and Fig. 4 showed a side chain structure of a critical amino acid in the sensor domain of the prediction structure of the BCP58, and three amino acids showing amino acid side chains respectively were S77, S81, and F82.

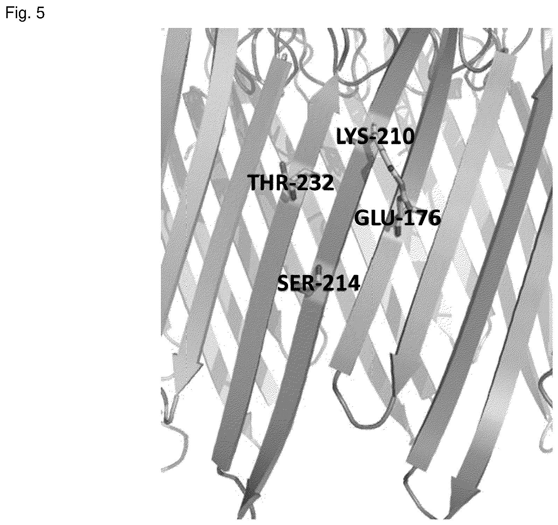

Amino acids in a porin transmembrane domain in a direction toward a membrane were more preferred to hydrophobic amino acids, and charged amino acids and polar amino acids might affect pore insertion efficiency. Fig. 5 showed four charged and polar amino acids in the BCP58 transmembrane domain in the direction toward the membrane, and the amino acids respectively were E176, K210, S214, and T232.

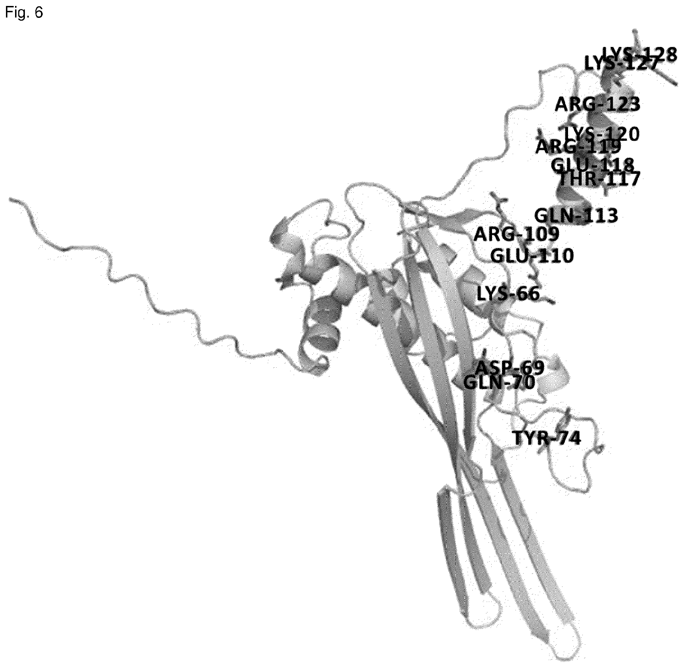

Since nucleic acids were negatively charged, if the amino acids at an entrance of a porin were positively charged, a library capture rate of nanopore sequencing might be increased, otherwise, the library capture rate might be reduced. Meanwhile, a binding strength of the porin also had a certain impact on a sequencing speed and sequencing current signal. Fig. 6 showed some critical amino acids at the entrance of the BCP58 monomer, and the amino acids respectively were K66, D69, Q70, Y74, R109, E110, Q113, T117, E118, R119, K120, R123, K127, and K128.

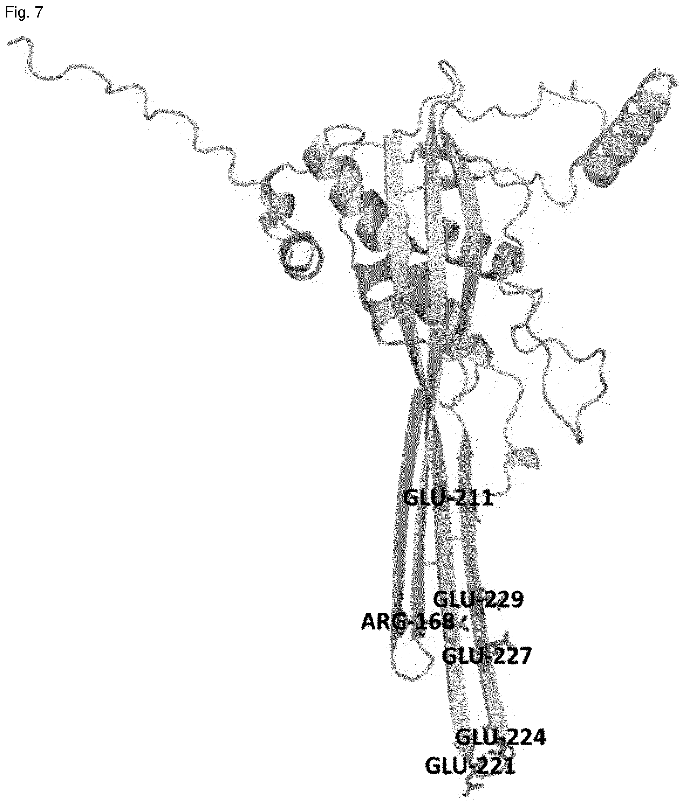

Furthermore, the smooth passing and leaving of an object to be tested from a nanopore were affected by some amino acids on an inner pore wall and entrance. Fig. 7 showed some amino acids on the inner pore wall and entrance of the BCP58 monomer that might affect the smooth passing of the object to be tested, and the amino acids respectively were R168, E211, E221, E224, E227, and E229.

### Embodiment 2 Construction of porin monomer and mutant expression vector thereof

Through an In-fusion method, *Nde*I and *Xho*I were used for enzyme digestion, and then a DNA sequence encoding the porin monomer (SEQ ID NO: 7) was inserted in a multiple cloning site of a vector pET24a. A Strepll amino acid was added on a C terminal of an amino acid sequence of the porin monomer (SEQ ID NO: 1) as a purification tag, and a filter tag was kanamycin. Through a site-directed mutation method, an Agilent site-directed mutagenesis kit was used, and an expression vector of the porin BCP58 monomer was used as a template, so as to construct corresponding mutants. In this embodiment, a Mutant 1 (S77A/F82Q), a Mutant 2 (S81N/F82A), a Mutant 3 (S81N/F82A/K120N), a Mutant 4 (S81N/F82A/K127S), and a Mutant 5 (E176I) were constructed. The Mutant 1 and the Mutant 2 were mutations in a gating domain; the Mutant 3 and the Mutant 4 were mutations in the gating domain and an entrance region; and the Mutant 5 was a mutation on an outer wall of the porin transmembrane domain. The method for constructing a mutant was the same as that for the Wild Type (WT).

### Embodiment 3 Culture and induction of porin monomer strain

The constructed porin monomer and a mutant expression plasmid thereof were respectively independently transformed into an *Escherichia coli* expression strain *E.coli* BL21(DE3), bacterial fluid was uniformly smeared on a plate containing 50 µg/mL of kanamycin, and culture was performed at 37 °C overnight. On the next day, a single colony was inoculated in a 5mL LB liquid culture medium containing 50 µg/mL of the kanamycin, and culture was performed at 37 °C and 200 rpm overnight. The obtained bacterial fluid was inoculated in a 50mL LB liquid culture medium containing 50 µg/mL of the kanamycin according to a volume ratio of 1:100, and culture was performed for 4 h at 37 °C and 200 rpm. The expanded and cultured bacterial fluid was inoculated in a 2L LB liquid culture medium containing 50 µg/mL of the kanamycin according to a volume ratio of 1:100, and culture was performed at 37 °C and 200 rpm. After an OD₆₀₀ value reached about 0.6-0.8, Isopropyl β-D-1-thiogalactopyranoside (IPTG) with a final concentration of 0.5 mM was added, and culture was performed for about 16-18 h at 16 °C and 200 rpm. The bacterial fluid was centrifuged and collected at 8000 rpm, and bacteria were frozen for storage at -20 °C for later use.

### Embodiment 4 Extraction and purification of recombinant porin monomer

### (1) Buffer preparation

Buffer A: 20 mM Tris-HCl, 250 mM NaCl, 1%DDM, pH 8.0.

Buffer B: 20 mM Tris-HCl, 250 mM NaCl, 0.05%DDM, pH 8.0.

Buffer C: 20 mM Tris-HCl, 250 mM NaCl, 0.05%DDM, 5 mM desthiobiotin, pH 8.0.

### (2) Purification step

The bacterial cells were fully resuspended at a ratio of 1 g of cells per 10 mL of Buffer A, and cells were broken through ultrasonic until the bacterial solution was clarified. Then, the solution was placed on a rotation instrument for rotation at 4 °C overnight. On the next day, centrifugation was performed for 1 h at 4 °C and 18000 rpm, supernatant was taken, filtration was performed with a 0.22 µm filter membrane, and then the solution was placed at 4 °C for later use.

Strep-Tactin beads (IBA Lifesciences) chromatography columns were equilibrated with the Buffer A using an AKTA pure chromatograph for 5 Column Volumes (CV), and then sample loading was performed at 2 mL/min. After sample loading, 20 CVs were washed with the Buffer B, the Buffer C was used for elution, and a target protein was collected.

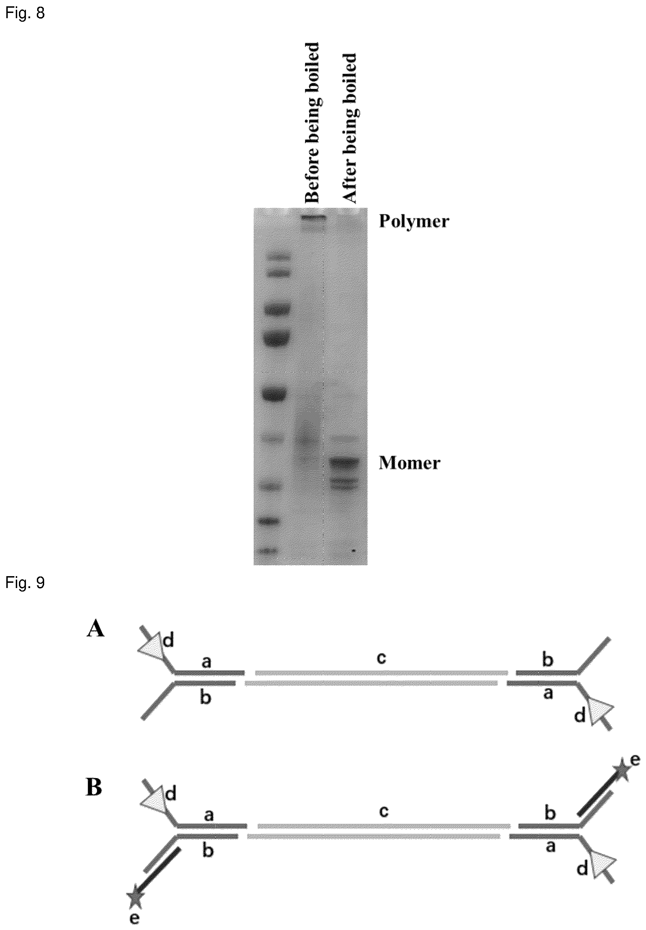

The obtained protein was concentrated to 1 mL, and then passed through a Superdex 6 increase 10/300GL(Cytiva) column equilibrated by the buffer B; and the target protein was collected, and then stored at -80 °C. SDS-PAGE was performed on the target protein obtained after purification, and results were shown in Fig. 8. Lanes 2 and 3 correspond to the electrophoretic bands of the non-denatured (BCP58 pore protein nonamer) and the denatured (after boiling at 95°C, resulting in pore protein monomers), respectively. The results show that the target protein is in a polymeric state under unboiled conditions and in a monomeric state after being boiled. SDS-PAGE results of the mutants were the same as the performance of a wild-type protein.

### Embodiment 5 Library construction

A sense strand and an antisense strand (SEQ ID NO: 15) of two partially regionally complementary DNA strands were annealed to form an adapter, and were connected to and purified with a double-stranded target fragment to be tested pUC57 (SEQ ID NO: 16) at room temperature with a T4 DNA ligase, so as to prepare a sequencing library. Then the sequencing library and a helicase BCH105 (SEQ ID NO: 17) were incubated for 1 h at 25 °C (a molar concentration ratio was 1:8), so as to form a sequencing library containing a BCH105 motor protein with a structure shown in A in Fig. 9. During sequencing, the sequencing library could further bound to a single-stranded DNA with cholesterol (SEQ ID NO: 18, the cholesterol was connected to a 5' terminal of the DNA), so as to form a structure shown in B in Fig. 9.

Adapter sequence sense strand: S1-(iSp18)₄-S2.

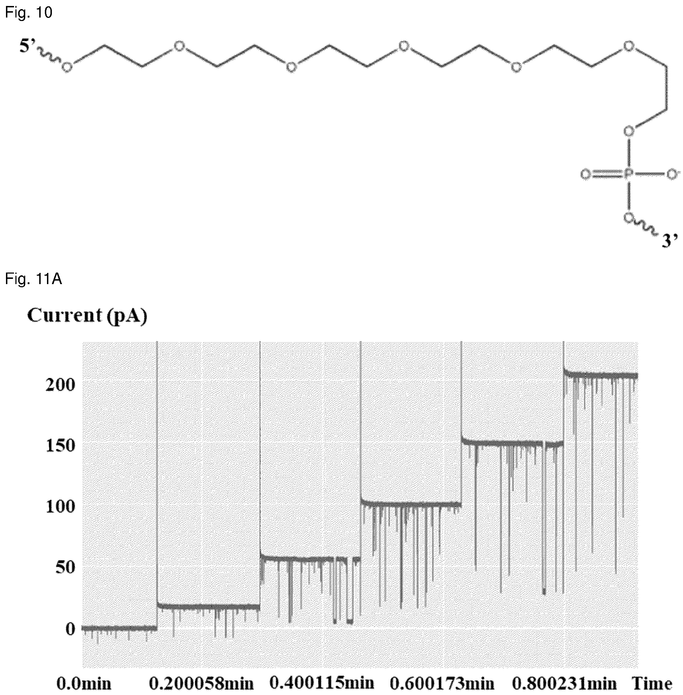

A sequence of S1 was shown in SEQ ID NO: 13, a sequence of S2 was shown in SEQ ID NO: 14, and a structure of iSp18 was shown in Fig. 10.
SEQ ID NO: 13: tttttttttttttttttttttttttttttttttttttttt.
SEQ ID NO: 14: ggttgtttctgttggtgctgatattgct.
SEQ ID NO: 15 (an antisense strand of the adapter sequence):
   gcaatatcagcaccaacagaaacaacctttgaggcgagcggtcaa.
SEQ ID NO: 16: 
tcgcgcgtttcggtgatgacggtgaaaacctctgacacatgcagctcccggagacggtcacagcttgtctgtaagcggatgccggg
agcagacaagcccgtcagggcgcgtcagcgggtgttggcgggtgtcggggctggcttaactatgcggcatcagagcagattgtactgag
agtgcaccatatgcggtgtgaaataccgcacagatgcgtaaggagaaaataccgcatcaggcgccattcgccattcaggctgcgcaact
gttgggaagggcgatcggtgcgggcctcttcgctattacgccagctggcgaaagggggatgtgctgcaaggcgattaagttgggtaacgc
cagggttttcccagtcacgacgttgtaaaacgacggccagtgaattcgagctcggtacctcgcgaatgcatctagatatcggatcccgggc
ccgtcgactgcagaggcctgcatgcaagcttggcgtaatcatggtcatagctgtttcctgtgtgaaattgttatccgctcacaattccacacaa
catacgagccggaagcataaagtgtaaagcctggggtgcctaatgagtgagctaactcacattaattgcgttgcgctcactgcccgctttcc
agtcgggaaacctgtcgtgccagctgcattaatgaatcggccaacgcgcggggagaggcggtttgcgtattgggcgctcttccgcttcctc
gctcactgactcgctgcgctcggtcgttcggctgcggcgagcggtatcagctcactcaaaggcggtaatacggttatccacagaatcagg
ggataacgcaggaaagaacatgtgagcaaaaggccagcaaaaggccaggaaccgtaaaaaggccgcgttgctggcgtttttccatag
gctccgcccccctgacgagcatcacaaaaatcgacgctcaagtcagaggtggcgaaacccgacaggactataaagataccaggcgttt
ccccctggaagctccctcgtgcgctctcctgttccgaccctgccgcttaccggatacctgtccgcctttctcccttcgggaagcgtggcgctttc
tcatagctcacgctgtaggtatctcagttcggtgtaggtcgttcgctccaagctgggctgtgtgcacgaaccccccgttcagcccgaccgctg
cgccttatccggtaactatcgtcttgagtccaacccggtaagacacgacttatcgccactggcagcagccactggtaacaggattagcaga
gcgaggtatgtaggcggtgctacagagttcttgaagtggtggcctaactacggctacactagaagaacagtatttggtatctgcgctctgctg
aagccagttaccttcggaaaaagagttggtagctcttgatccggcaaacaaaccaccgctggtagcggtggtttttttgtttgcaagcagca
gattacgcgcagaaaaaaaggatctcaagaagatcctttgatcttttctacggggtctgacgctcagtggaacgaaaactcacgttaaggg
attttggtcatgagattatcaaaaaggatcttcacctagatccttttaaattaaaaatgaagttttaaatcaatctaaagtatatatgagtaaactt
ggtctgacagttaccaatgcttaatcagtgaggcacctatctcagcgatctgtctatttcgttcatccatagttgcctgactccccgtcgtgtaga
taactacgatacgggagggcttaccatctggccccagtgctgcaatgataccgcgagacccacgctcaccggctccagatttatcagcaa
taaaccagccagccggaagggccgagcgcagaagtggtcctgcaactttatccgcctccatccagtctattaattgttgccgggaagcta
gagtaagtagttcgccagttaatagtttgcgcaacgttgttgccattgctacaggcatcgtggtgtcacgctcgtcgtttggtatggcttcattca
gctccggttcccaacgatcaaggcgagttacatgatcccccatgttgtgcaaaaaagcggttagctccttcggtcctccgatcgttgtcagaa
gtaagttggccgcagtgttatcactcatggttatggcagcactgcataattctcttactgtcatgccatccgtaagatgcttttctgtgactggtga
gtactcaaccaagtcattctgagaatagtgtatgcggcgaccgagttgctcttgcccggcgtcaatacgggataataccgcgccacatagc
agaactttaaaagtgctcatcattggaaaacgttcttcggggcgaaaactctcaaggatcttaccgctgttgagatccagttcgatgtaaccc
actcgtgcacccaactgatcttcagcatcttttactttcaccagcgtttctgggtgagcaaaaacaggaaggcaaaatgccgcaaaaaagg
gaataagggcgacacggaaatgttgaatactcatactcttcctttttcaatattattgaagcatttatcagggttattgtctcatgagcggataca
 
tatttgaatgtatttagaaaaataaacaaataggggttccgcgcacatttccccgaaaagtgccacctgacgtctaagaaaccattattatca
tgacattaacctataaaaataggcgtatcacgaggccctttcgtc.
SEQ ID NO: 17:

MKHDLSSATEEQRYIFTDFIKQAQLALKGDSDYEVFAIQGFAGSGKTWLSALIIDELLELGMKVA
VTSPTHKAVRVSLNMLKNNGIDTNSPLMYPGTIHHFLNLKLDHGFADDGTACNVTTKAKLVVNKFN
EALEYVDVLIVDEASMVSGELYDHALKTLGDRAKIILFIGDSYQLLPVDDEDSSIFLKDDIFHYKLTKV
VRQAEDNIIIAKSQELIKAMDQKTYYPSVNDYFVNITEDTEGIKLLKSNVELFELYFSDFKDKMTGAY
TNKVVNQFNEYIRYTLYQETKFIADKDELVFQETYTDSKGNIIVSNGEIIEVATAKLTTDIDKFKIWKIV
SKKNELGEAVRFNVLDPSSYNEFNDLLDKYCADAKIAKGYDRSKAWKKYFKLKEKYAKVRYNFSS
TIHKLQGSTYQNMYFDMRGLDYFYRMNRDNVLRLVYVGITRASDQVFILQD.

A sequence of the single-stranded DNA with cholesterol:
cholesterol - ttgaccgctcgcctc (SEQ ID NO: 18).

### Embodiment 6 Construction of nanopore biosensor using porin BCP58 and mutant thereof

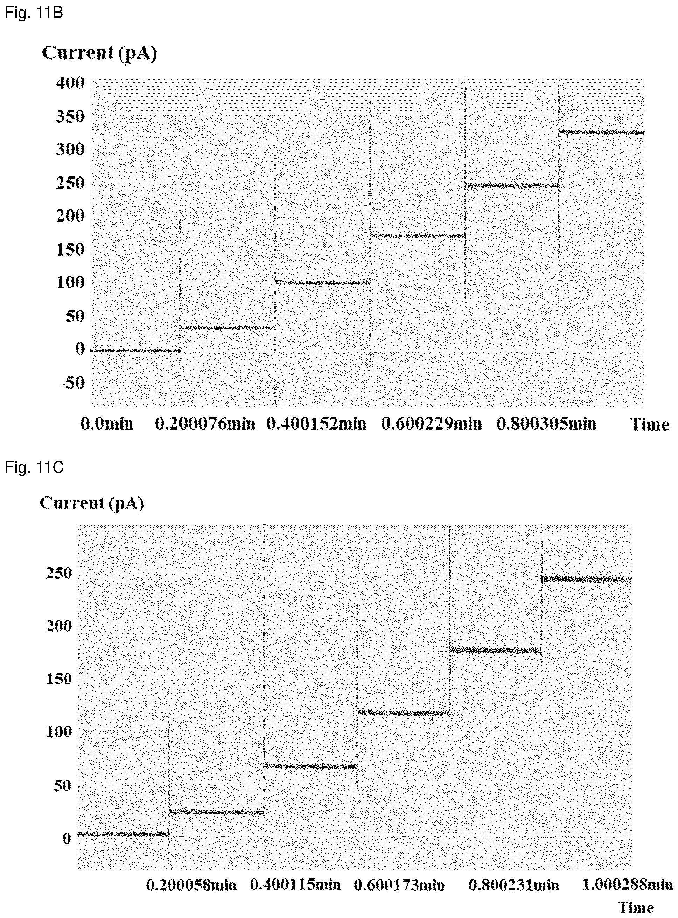

A current signal was collected with a patch clamp amplifier. A single channel nanopore detection system based on a patch clamp and a signal amplifier was built according to a method disclosed in a document (Ji Z, Guo P. Channel from bacterial virus T7 DNA packaging motor for the differentiation of peptides composed of a mixture of acidic and basic amino acids.Biomaterials.2019 May 21; 214:119222). Ag/AgCl electrodes were soaked in a sequencing buffer, and the electrodes respectively were located in a cis region and a trans region of an electrolytic tank. A 1xPBS buffer was used to dilute the porin (i.e., the porin BCP58 obtained through purification in Embodiment 4) to a certain number of times, and then the single nanopore protein BCP58 was inserted into a phospholipid bilayer consisting of DPhPC under the action of an externally applied electric field force, so as to form the nanopore biosensor. Dilution times were based on whether the porin is embedded in a membrane (i.e., an embedding pore). Generally, a protein concentration of 0.1 mg/mL was used, and the PBS was used to dilute 100x or 50x or other times for attempt. If some dilution failed to embed the pore, the dilution times were required to be reduced for continuous attempt, until the nanopore protein was successfully embedded in the membrane layer. An external voltage was applied to obtain a current amplitude value of the single porin. Fig. 11A showed biosensing currents generated by a pore channel of the nanopore protein BCP58 WT when voltages of 0.02 V, 0.04 V, 0.10 V, 0.14 V, and 0.18 V were applied. Fig. 11B showed biosensing currents generated by a pore channel of the nanopore protein BCP58 mutant 1 when voltages of 0.02 V, 0.04 V, 0.10 V, 0.14 V, and 0.18 V were applied. Fig. 11C showed biosensing currents generated by a pore channel of the nanopore protein BCP58 mutant 2 when voltages of 0.02 V, 0.04 V, 0.10 V, 0.14 V, and 0.18 V were applied.

It might be seen that, the mutant 1 and the mutant 2 might significantly reduce open-pore current noise compared to the WT. Therefore, mutants with three amino acid mutations, which were S77, S81, and F82, in the gating domain had the potential to reduce the open-pore current noise of the BCP58 porin at different voltages.

### Embodiment 7 DNA sequencing using porin BCP58 and mutant thereof

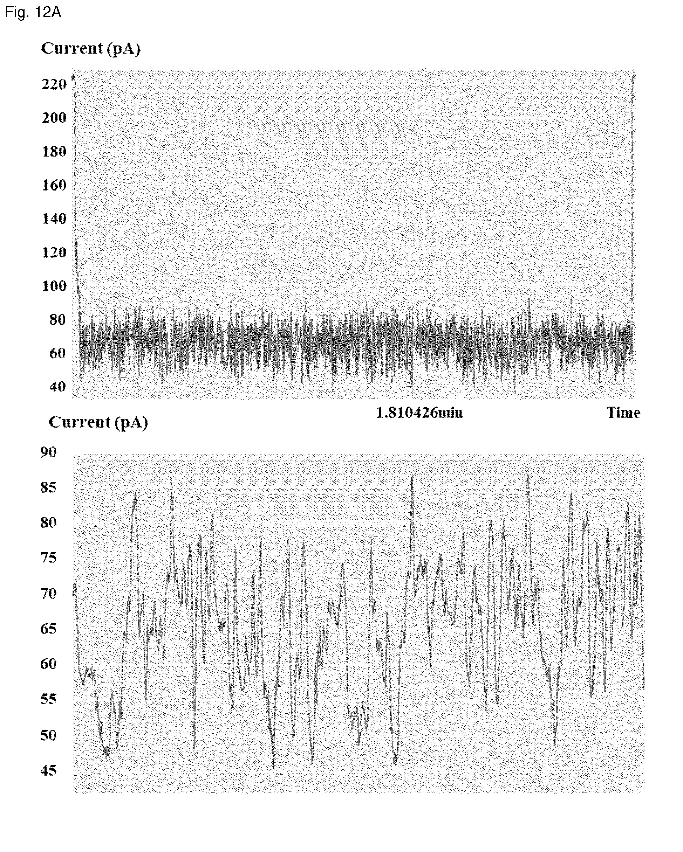

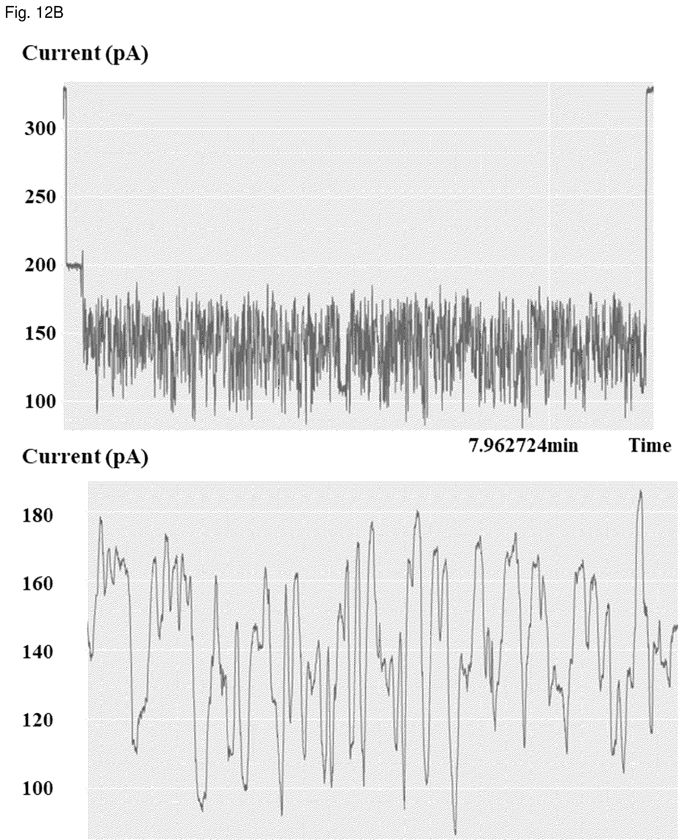

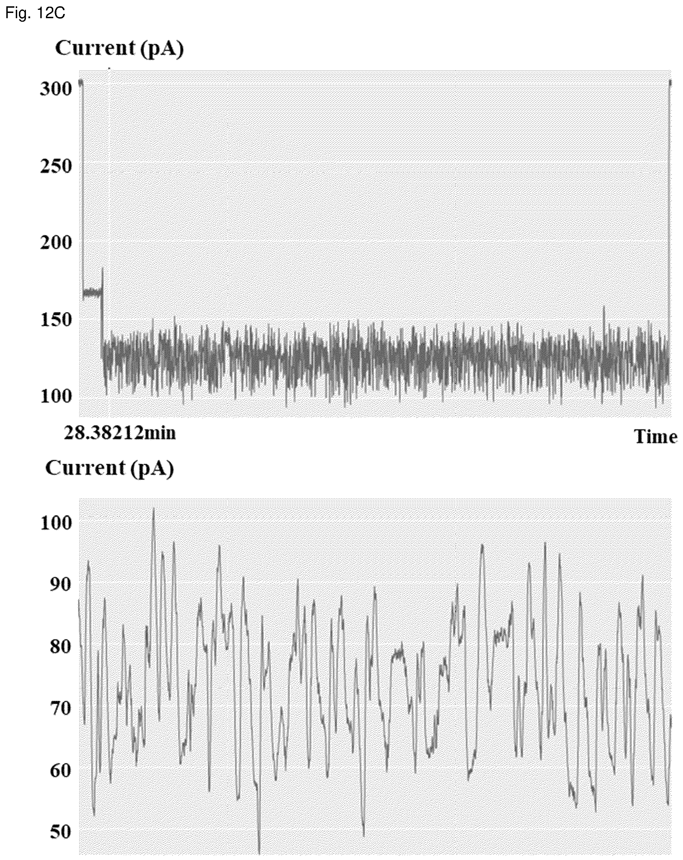

The sequencing library (SEQ ID NO: 16) containing a pUC57 sequence prepared in Embodiment 5 and the single-stranded DNA with cholesterol (SEQ ID NO: 18, the cholesterol was connected to the 5' terminal of the DNA) were mixed with the sequencing buffer, and added to the nanopore biosensor. After the external voltage of 0.14 V or 0.18 V was applied, the DNA was observed to be captured by the nanopore, and a characteristic blocking current amplitude value was generated. Furthermore, as the DNA moved by passing through the nanopore, the current amplitude value varied. Different DNA sequences generated different blocking current amplitude values. The single-stranded DNA with cholesterol might bind to the phospholipid bilayer, facilitating the capturing of the sequencing library by the nanopore, thereby reducing a sample size of the sequencing library. Fig. 12A showed current changes generated when library DNA passed through the nanopore protein BCP58 WT and local details under the action of the external voltage of 0.18 V. Fig. 12B showed current changes generated when library DNA passed through the nanopore protein BCP58 mutant 1 and local details under the action of the external voltage of 0.18 V. Fig. 12C showed current changes generated when library DNA passed through the nanopore protein BCP58 mutant 2 and local details under the action of the external voltage of 0.18 V.

It might be seen that, at the external voltage of 0.18 V, a sequencing amplitude of the nanopore protein BCP58 WT was about 40 pA, a sequencing amplitude of the mutant 1 was about 80 pA, and a sequencing amplitude of the mutant 2 was about 50 pA.

Sequencing buffer: 0.47 M KCI, 25 mM HEPES, 1 mM EDTA, 5 mM ATP, 25 mM MgCl₂, pH7.6.

### Embodiment 8 Study on library capture by porin BCP58 and mutant thereof

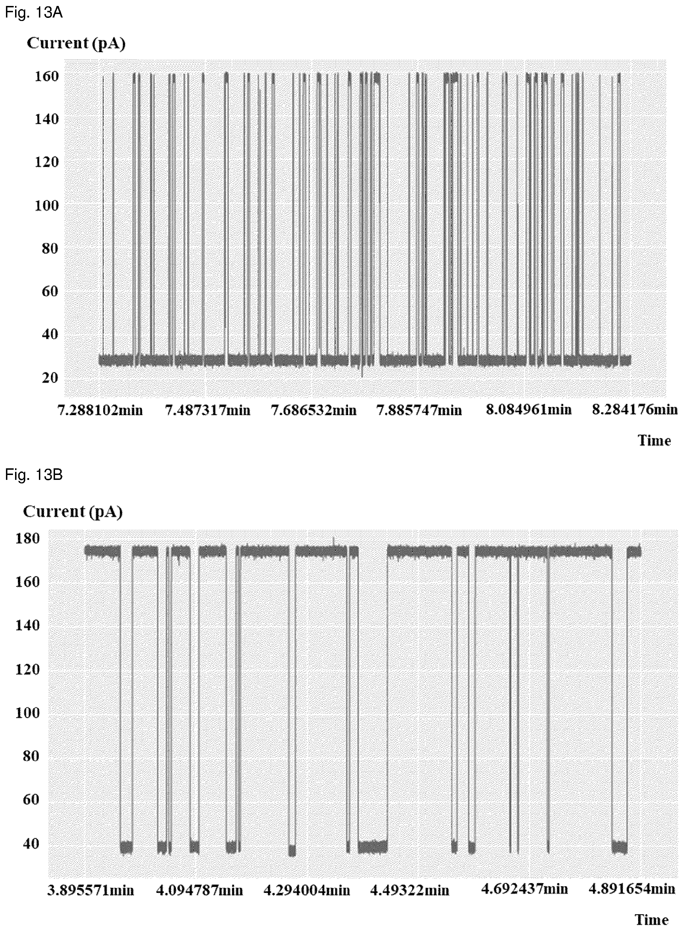

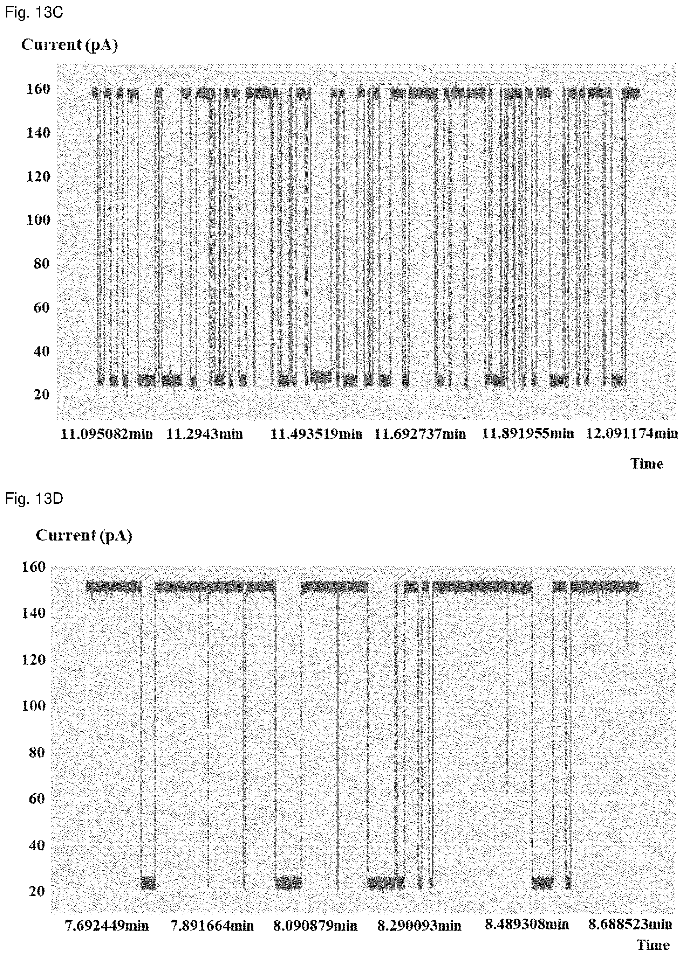

Nanopore embedding was performed on the nanopore protein BCP58 WT, the Mutant 3, the Mutant 4, and a porin CSGG-Y51A/F56Q in the prior art, respectively; and after pore embedding was completed, single pores were selected according to the open-pore current. 3 µL of 10 µM QC DNA (SEQ ID NO: 19) was added to the cis region of the electrolytic tank. The QC DNA might form a G4 structure, and under the action of the electric field force, the G4 structure might be pulled apart. After the external voltage of 0.14 V was applied, the QC DNA was observed to be captured by the nanopore, so as to generate a blocking current; and the QC DNA completely passed through the nanopore, then the current returned to the open-pore current, so as to capture new QC DNA. Fig. 13A showed a capture effect for the QC DNA by the nanopore protein BCP58 WT at a voltage of 0.14 V. Fig. 13B showed a capture effect of the QC DNA by the nanopore protein BCP58 mutant 3 at a voltage of 0.14 V. Fig. 13C showed a capture effect for the QC DNA by the nanopore protein BCP58 mutant 4 at a voltage of 0.14 V. Fig. 13D showed a capture effect for the QC DNA by the nanopore protein CSGG-Y51A/F56Q in the prior art at a voltage of 0.14 V.

It might be seen that, the capture capabilities for the QC DNA by the BCP58 WT and Mutant 4 were significantly better than the porin CSGG-Y51A/F56Q in the prior art.

The BCP58 mutants 3 and 4, which contain mutations targeting positively charged residues at the entrance, had varying degrees of reduced capture capabilities for the QC DNA compared to WT.

In view of the strong capture capability of the library by the porin BCP58, compared to the prior art, with the porin for nanopore sequencing, the porin had the potential to increase sequencing throughput and reduce the sample size of the library.

QC DNA (SEQ ID NO: 19): ttttttttttggtggtgtggttgg.

From the above descriptions, it might be seen that, the embodiments of the present disclosure implemented the following technical effects. The present disclosure discovered a new nanopore protein monomer. The monomer became the pore BCP58 by means of polymerization. The porin and the mutant thereof had the potential to increase sequencing throughput and reduce the sample size of the library. For example, the mutations of the three amino acids S77, S81, and S82 in the gating domain might effectively reduce sequencing noise and increase the sequencing amplitude, and the mutations of some amino acids at the entrance had significant capability of capturing a DNA library. The nanopore protein and the mutant thereof of the present disclosure were good in stability, and could meet requirements for nanopore sequencing.

The above are only the preferred embodiments of the present disclosure and are not intended to limit the present disclosure. For those skilled in the art, the present disclosure may have various modifications and variations. Any modifications, equivalent replacements, improvements and the like made within the spirit and principle of the present disclosure all fall within the scope of protection of the present disclosure.

## Claims

1. A porin monomer, wherein the porin monomer comprises:
(a) a protein having an amino acid sequence shown in SEQ ID NO: 1; or
(b) a protein mutant, wherein an amino acid sequence of the protein mutant is subjected to substitution, deletion and/or addition of one or more amino acids at at least one of the following sites in SEQ ID NO: 1: 77, 81, 82, 176, 210, 214, 232, 66, 69, 70, 74, 109, 110, 113, 117, 118, 119, 120, 123, 127, 128, 168, 211, 221, 224, 227, and 229, and the mutant has a function of forming a pore channel structure by means of polymerization; or
(c) a protein having at least 70%, 75%, 80%, 85%, 90%, 95%, or 99% homology to the protein in the (a) or the (b), and the protein has the function of forming the pore channel structure by means of polymerization.

2. The porin monomer according to claim 1, wherein in the (b), types of amino acids substituted are each independently selected as follows:
S77 is mutated into S77G, S77A, or S77T;
S81 is mutated into S81G, S81A, S81T, S81N, or S81Q;
F82 is mutated into F82G, F82A, F82S, F82T, F82N, or F82Q;
E176 is mutated into E176A, E176G, E176V, E176L, E176I, E176Y, E176F, or E176W;
K210 is mutated into K210A, K210G, K210V, K210L, K210I, K210Y, K210F, or K210W;
S214 is mutated into S214A, S214G, S214V, S214L, S214I, S214Y, S214F, or S214W;
T232 is mutated into T232A, T232G, T232V, T232L, T232I, T232Y, T232F, or T232W;
K66 is mutated into K66N, K66A, K66G, K66S, K66T, or K66Q;
D69 is mutated into D69K, D69R, D69N, D69A, D69G, D69S, D69T, or D69Q;
Q70 is mutated into Q70K, Q70R, Q70D, Q70E, Q70N, Q70A, Q70G, Q70S, Q70T, or Q70Q;
Y74 is mutated into Y74N, Y74A, Y74G, Y74S, Y74T, or Y74Q;
R109 is mutated into R109N, R109A, R109G, R109S, R109T, or R109Q;
E110 is mutated into E110K, E110R, E110N, E110A, E110G, E110S, E110T, or E110Q;
Q113 is mutated into Q113K, Q113R, Q113N, Q113A, Q113G, Q113S, or Q113T;
T117 is mutated into T117K, T117R, T117N, T117A, T117G, T117S, or T117Q;
E118 is mutated into E118K, E118R, E118N, E118A, E118G, E118S, E118T, or E118Q;
R119 is mutated into R119N, R119A, R119G, R119S, R119T, or R119Q;
K120 is mutated into K120N, K120A, K120G, K120S, K120T, or K120Q;
R123 is mutated into R123N, R123A, R123G, R123S, R123T, or R123Q;
K127 is mutated into K127N, K127A, K127G, K127S, K127T, or K127Q;
K128 is mutated into K128N, K128A, K128G, K128S, K128T, or K128Q;
R168 is mutated into R168N, R168Q, R168S, R168T, R168A, or R168G;
E211 is mutated into E211N, E211Q, E211S, E211T, E211A, or E211G;
E221 is mutated into E221N, E221Q, E221S, E221T, E221A, or E221G;
E224 is mutated into E224N, E224Q, E224S, E224T, E224A, or E224G;
E227 is mutated into E227N, E227Q, E227S, E227T, E227A, or E227G; and
E229 is mutated into E229N, E229Q, E229S, E229T, E229A, or E229G.

3. The porin monomer according to claim 1 or 2, wherein in the (b), the types of amino acids substituted are each independently selected as follows: S77A, F82Q, S81N, F82A, K120N, K127S, and E176I.

4. The porin monomer according to any one of claims 1 to 3, comprising a protein having an amino acid sequence in any one of SEQ ID NO: 2 to SEQ ID NO: 6.

5. A protein construct, wherein the protein construct is formed by 2 or more porin monomers according to any one of claims 1 to 4 through a covalent or non-covalent linkage.

6. A porin, wherein the porin is formed by 7-11 of the porin monomers according to any one of claims 1 to 4 through a covalent or non-covalent linkage, preferably formed by 9 of the porin monomers.

7. The porin according to claim 6, wherein the porin is formed by 9 of the porin monomers through a non-covalent linkage, wherein the porin monomer comprises a protein having an amino acid sequence in any one of SEQ ID NO: 1 to SEQ ID NO: 6.

8. The porin according to claim 6 or 7, wherein a pore channel diameter of the porin is 0.5-3.0 nm.

9. A kit, wherein the kit comprises the porin monomer according to any one of claims 1 to 4, or the protein construct according to claim 7, or the porin according to any one of claims 6 to 8.

10. The kit according to claim 9, wherein the kit further comprises a membrane layer, wherein the membrane layer comprises a lipid layer or an artificial polymer membrane.

11. The kit according to claim 10, wherein the kit further comprises at least one of the following: a sequencing buffer, a nuclease, a polymerase, a topoisomerase, a ligase, a helicase, or a cholesterol-linked single-stranded DNA.

12. An isolated DNA molecule, wherein the DNA molecule comprises:
a nucleotide sequence encoding the porin monomer according to any one of claims 1 to 4, or a nucleotide sequence encoding the protein construct according to claim 7, or a nucleotide sequence encoding the porin according to any one of claims 6 to 8.

13. The DNA molecule according to claim 12, having more than 70%, preferably more than 80%, more preferably more than 90%, further preferably more than 95%, and most preferably more than 99% homology to an nucleotide sequence in any one of SEQ ID NO: 7 to SEQ ID NO: 12 and encoding a protein having the same function.

14. A recombinant vector, wherein the recombinant vector comprises the DNA molecule according to claim 12 or 13.

15. A host cell, wherein the host cell is transformed in the recombinant vector according to claim 14.

16. A nanopore sensor, wherein the nanopore sensor comprises:
a membrane layer; and
a porin inserted in the membrane layer and forming a pore channel, wherein the pore channel generates a current when a voltage is applied across the membrane layer; and
the porin comprises the porin according to any one of claims 6 to 8.

17. The nanopore sensor according to claim 16, wherein the membrane layer comprises a lipid layer or an artificial polymer membrane;
preferably, the lipid layer comprises an amphiphilic lipid;
preferably, the amphiphilic lipid comprises a phospholipid bilayer;
preferably, the lipid layer comprises a planar membrane layer or a liposome;
preferably, the liposome comprises a multi-layer liposome or a single-layer liposome; and
preferably, the lipid layer comprises a phospholipid bilayer consisting of 1,2-diphytanoyl-sn-glycero-3-phosphocholine (DPhPC).

18. The nanopore sensor according to claim 16, wherein when a voltage is applied across the membrane layer, a biomolecule to be tested passes through the pore channel in the nanopore sensor and undergoes displacement, and the pore channel generates a changing current;
preferably, the biomolecule to be tested comprises DNA, RNA, or peptide; and
preferably, the DNA and/or RNA comprises any one or more of the following modified bases: 5-methylcytosine, 6-methyladenine, 7-methylguanine, and pseudouridine.

19. A nanopore sequencing apparatus, wherein the nanopore sequencing apparatus comprises the nanopore sensor according to any one of claims 16 to 18.

20. The nanopore sequencing apparatus according to claim 19, wherein the nanopore sequencing apparatus comprises:
an electrolytic tank, comprising a sequencing buffer;
a nanopore sensor, located in the center of the electrolytic tank, and dividing the electrolytic tank and the sequencing buffer into a positive electrode electrolyte region and a negative electrode electrolyte region; and
a first electrode and a second electrode, respectively disposed in the positive electrode electrolyte region and the negative electrode electrolyte region, and both connected to a signal processing chip, wherein
preferably, the first electrode and the second electrode are made of metals or composite electrode materials; and
preferably, the first electrode and the second electrode are different, and respectively are made of silver and silver chloride; or the first electrode and the second electrode are the same, and are made of gold, platinum, graphene, or titanium nitride.

21. A sequencing method, wherein the sequencing method uses the porin according to any one of claims 6 to 8, or the nanopore sensor according to any one of claims 16 to 18, or the nanopore sequencing apparatus according to claim 19 or 20 to detect and analyze an electrical signal generated when a biomolecule to be tested passes through a pore channel of the porin, and determine a sequence of the biomolecule to be tested.

22. The sequencing method according to claim 21, wherein the biomolecule to be tested comprises modified or unmodified DNA, RNA, or peptide; and
preferably, the electrical signal comprises a current.

23. The sequencing method according to claim 21, wherein the biomolecule to be tested is a target nucleotide sequence, and the sequencing method comprises:
(a) causing the target nucleotide sequence to be in contact with the porin according to any one of claims 6 to 8 and a nucleic acid binding protein, the nucleic acid binding protein controls a movement speed of the target nucleotide sequence passing through the pore channel of the porin, wherein the nucleic acid binding protein is selected from any one or more of a nuclease, a polymerase, a topoisomerase, a ligase, a helicase, and a single-strand binding protein; and
(b) when applying a voltage across the pore channel and the target nucleotide sequence moves and passes through the pore channel, measuring an electrical signal passing through the pore channel, wherein the electrical signals generated by different types of nucleotides passing through the pore channel are different, thereby determining sequence information of the target nucleotide sequence based on the electrical signals.

24. An application of the porin monomer according to any one of claims 1 to 4, or the porin according to any one of claims 6 to 8, or the kit according to any one of claims 9 to 11, or the DNA molecule according to claim 12 or 13, or the recombinant vector according to claim 14, or the host cell according to claim 15, or the nanopore sensor according to any one of claims 16 to 18, or the nanopore sequencing apparatus according to claim 19 or 20 in biological small molecule detection, nucleic acid sequencing, or peptide sequencing.
